# EUROPÄISCHE PATENTSCHRIFT

(11) **EP 1 423 525 B1**
(45) Veröffentlichungstag und Bekanntmachung des Hinweises auf die Patenterteilung: **30.07.2008**
(21) Anmeldenummer: 02797955.8
(22) Anmeldetag: 05.09.2002
(51) Int. Cl.: C12N 15/863, C12N 15/39, C12N 15/51, C12N 5/10, A61K 39/285, A61K 39/29

(54) **REKOMBINANTES MVA MIT DER FÄHIGKEIT ZUR EXPRESSION VON HCV STRUKTUR-ANTIGENEN**
RECOMBINANT MVA CAPABLE OF EXPRESSING STRUCTURAL HCV ANTIGENS
MVA DE RECOMBINAISON CAPABLE D'EXPRIMER DES ANTIGENES STRUCTURELS DU VIRUS DE L'HEPATITE C

(30) Priorität: 05.09.2001 DE 10143490
(43) Veröffentlichungstag der Anmeldung: 02.06.2004
(73) Patentinhaber: Helmholtz Zentrum München Deutsches Forschungszentrum für Gesundheit und Umwelt (GmbH), 85764 Neuherberg (DE)
(72) Erfinder: SUTTER, Gerd, 80803 München (DE); ERFLE, Volker, 81679 München (DE); STAIB, Caroline, 80804 München (DE); WANG, Yuan,, Shanghai200233 (CN); LI, Guangdi,, Shanghai 200233 (CN); ZHU, Lixin,, Shanghai 200031 (CN)
(74) Vertreter: Behnisch, Werner
(86) Internationale Anmeldenummer: PCT/EP2002/009958
(87) Internationale Veröffentlichungsnummer: WO 2003/023042

(56) Entgegenhaltungen:
- WO-A-00/26385
- ANTOINE G ET AL: "THE COMPLETE GENOMIC SEQUENCE OF THE MODIFIED VACCINIA ANKARA STRAIN: COMPARISON WITH OTHER ORTHOPOXVIRUSES" VIROLOGY, ACADEMIC PRESS,ORLANDO, US, Bd. 244, 1998, Seiten 365-396, XP000887332 ISSN: 0042-6822
- "TRANSIENT HOST RANGE SELECTION FOR GENETIC ENGINEERING OF MODIFIED VACCINIA VIRUS ANKARA" BIOTECHNIQUES, EATON PUBLISHING, NATICK, US, Bd. 28, Nr. 6, Juni 2000 (2000-06), Seiten 1137-1148, XP001026101 ISSN: 0736-6205
- RALSTON R ET AL: "CHARACTERIZATION OF HEPATITIS C VIRUS ENVELOPE GLYCOPROTEIN COMPLEXES EXPRESSED BY RECOMBINANT VACCINIA VIRUSES" JOURNAL OF VIROLOGY, NEW YORK, US, US, Bd. 67, Nr. 11, 1. November 1993 (1993-11-01), Seiten 6753-6761, XP000561254 ISSN: 0022-538X
- CHOO Q-L ET AL: "VACCINATION OF CHIMPANZEES AGAINST INFECTION BY THE HEPATITIS C VIRUS" PROCEEDINGS OF THE NATIONAL ACADEMY OF SCIENCES OF USA, NATIONAL ACADEMY OF SCIENCE. WASHINGTON, US, Bd. 91, Nr. 4, 15. Februar 1994 (1994-02-15), Seiten 1294-1298, XP002017755 ISSN: 0027-8424
- MOSS BERNARD: "Genetically engineered poxviruses for recombinant gene expression, vaccination, safety" PROCEEDINGS OF THE NATIONAL ACADEMY OF SCIENCES OF THE UNITED STATES, NATIONAL ACADEMY OF SCIENCE, WASHINGTON, DC, US, Bd. 93, Nr. 21, 1996, Seiten 11341-11348, XP002173270 ISSN: 0027-8424
- LIU J ET AL: "Expression, purification, immunological characterization and application of Escherichia coli-derived hepatitis C virus E2 proteins." BIOTECHNOLOGY AND APPLIED BIOCHEMISTRY. ENGLAND OCT 2001, Bd. 34, Nr. Pt 2, Oktober 2001 (2001-10), Seiten 109-119, XP002227004 ISSN: 0885-4513

## Beschreibung

Die Erfindung betrifft rekombinantes Modifiziertes Vaccinia Virus Ankara (MVA), welches in der Lage ist, HCV-Struktur-Antigene, immunogene Abschnitte dieser Struktur-Antigene oder Epitope dieser Struktur-Antigene zu exprimieren. Die Erfindung beschreibt weiterhin eine pharmazeutische Zubereitung, die insbesondere in Form einer Vakzine vorliegt und ein rekombinantes MVA gemäß der Erfindung enthält, Eukaryontenzellen, die das erfindungsgemäße rekombinante MVA enthalten sowie verschiedene Verwendungen des rekombinanten MVA, beispielsweise zur Produktion rekombinanter Strukturproteine, zur Herstellung einer pharmazeutischen Zubereitung, die insbesondere zur Therapie und Prophylaxe von HCV-Infektionen und hierdurch verursachter Erkrankungen geeignet ist, Verfahren zur Herstellung von rekombinantem MVA und von rekombinanten HCV-Struktur-Polypeptiden, die von diesem rekombinanten MVA kodiert werden, sowie DNA oder RNA dieses rekombinanten MVA.

### Einleitung

Das Hepatitis-C-Virus (HCV) ist ein positiv-strängiges RNA-Virus, das zur Familie Flaviviridae gehört. Es ist das hauptsächliche Agens einer nach einer Transfusion und in der Bevölkerung erworbenen Non-A-, Non-B-Hepatitis (8, 35). Mehr als 70% der infizierten Patienten entwickeln eine chronische Hepatitis mit dem Risiko, dass sich diese zur Zirrhose und zum hepatozellulären Karzinom weiter entwickelt (30, 53). Die gängigen Behandlungsmethoden sind eingeschränkt (39, 40, 31, 24, 13). Es wurden zwar viele Mühen in die Impfstoffentwicklung investiert, und es wurden auch einige ermutigende Ergebnisse erzielt (9, 41, 22, 25), jedoch ist bisher noch kein wirksamer und/oder therapeutischer Impfstoff verfügbar.

Die HCV-Strukturproteine, d.h. der virale Capsidprotein-Kern, und die Hüllglycoproteine E1 und E2 sind vielversprechende Impfstoff-Ziele: Das Core-Antigen ist bei verschiedenen HCV-Genotypen und -Subtypen hoch konserviert (6). Innere Antigene, wie Nukleokapside, gehen in verschiedenen Modellen, bspw. Rabies-Virus, Hepatitis-B-Virus und Influenza-Virus, mit schützenden Immunantworten einher (14, 33, 62). Mit den folgenden verschiedenen Ansätzen konnten HCV-Kern-spezifische Antikörper- und CTL-Antworten induziert und nachgewiesen werden (23, 32). Das Core-Protein scheint dagegen viele regulatorische Funktionen zu besitzen, die an der Modulation der Wirtszell-Apoptose, Transkription, Transformation und Immun-Präsentation beteiligt sind (43, 4). Die Immunisierung von Schimpansen mit rekombinanten E1- und E2-Proteinen induziert einen Schutz gegen einen homologen Challenge vermutlich über die Induktion neutralisierender Antikörper (9). Einer Anzahl von Berichten zufolge könnte die hypervariable Domäne (HVR), die sich am N-Terminus von E2 befindet, eine wichtige Neutralisierungsstelle sein (20, 34, 67, 68).

Das Fehlen eines geeigneten Tiermodells erschwert das Studium von Wirts-Immunreaktionen gegen HCV-Infektion und die Bewertung des Impfschutzes erheblich. Das Fehlen eines effizienten In-vitro-Zellkultursystems für eine produktive HCV-Vermehrung und geringe Mengen HCV-Teilchen in infizierten Lebergeweben oder Blut behinderten die Erzeugung und die Bewertung konventionellerer Impfstoffe, bspw. auf der Grundlage lebender abgeschwächter oder inaktivierter Viren. Die Verwendung von Vektor-Impfstoffen auf der Basis lebender Trägerviren zur Expression antigencodierender Sequenzen bietet einen alternativen Ansatz bei der HCV-Impfstoffentwicklung.

Es ist eine Aufgabe der vorliegenden Erfindung, ein neues Mittel bereitzustellen, welches zur Prophylaxe und Therapie von Mensch und Tier egen HCV-Infektionen geeignet ist und die oben beschriebenen Nachteile vermeidet.

Diese Aufgabe wird erfindungsgemäß durch rekombinantes MVA gemäß den Merkmalen des Anspruchs 1 gelöst, welches so modifiziert, wurde, daß es zur Expression von HCV-Struktur-Antigenen, immunogenen Abschnitten von HCV-Struktur-Antigenen und/oder von Epitopen dieser HCV-Struktur-Antigene, bevorzugt von Oberflächenepitopen, in der Lage ist.

Bevorzugte Ausgestaltungen der Erfindung sind aus der nachfolgenden Beschreibung sowie den beiliegenden Patentansprüchen zu entnehmen.

Erfindungsgemäß konnte gezeigt werden, daß rekombinantes MVA mit DNA-Sequenzen, die für HCV-Struktur-Antigene, immunogene Abschnitte hiervon oder für Epitope dieser HCV-Struktur-Antigene kodieren, zur Herstellung einer Vakzine hervorragend geeignet ist. Dies war deshalb überraschend, weil insbesondere die Produktion bzw. die Co-Produktion des HCV Core-Antigens die Immunogenität des Impfstoffs nicht beeinträchtigt. Die Veröffentlichung (19) zeigte nämlich, daß durch die Produktion des Core-Antigens durch ein replikationskompetentes Vaccinia-Virus die Immunantwort in einem infizierten bzw. geimpften Wirt in spezifischer Weise supprimiert wurde. Man konnte deshalb davon ausgehen, daß auch bei Verwendung von MVA als Vektor zur Produktion von HCV-Struktur-Antigenen eine derartige Immunsuppression erfolgt. Unerwarteterweise konnte aber gezeigt werden, daß bei Verwendung von MVA-Vektoren diese Immunsuppression wieder aufgehoben wird. Dieses erstaunliche, experimentell gestützte Resultat bietet eine hervorragende Basis zur Entwicklung von Impfstoffen, insbesondere auf der Grundlage des HCV-Antigens als Impfantigen.

Der erfindungsgemäß eingesetzte virale Vektor basiert auf dem modifizierten Vaccinia-Virus Ankara (MVA), einem stark abgeschwächten Vaccinia-Virus-Stamm, der sich höchstwahrscheinlich aufgrund seiner Replikationsdefizienz in Säugetier-Wirten für die klinische Verwendung als sicher erwiesen hat (42, 45, 60, 50). Rekombinantes MVA (rMVA) als Impfstoff erwies sich als wirksam bei der Anregung von T-Zell- oder Antikörperantworten auf verschiedene heterologe Antigene und als schützend beim Test in Tier-Challenge-Modellen für Infektionskrankheiten beim Menschen, einschließlich Influenza, AIDS, Masern und Malaria (61, 3, 27, 52, 15, 59, 1).

Trotz der an sich bekannten Verwendung von rekombinantem MVA zur Herstellung einer Vakzine war somit im vorliegenden Fall die Verwendung in Verbindung mit HCV-Struktur-Antigenen, nämlich dem Core-Antigen von HCV sowie E1 und E2, nicht nahegelegen.

Die erfindungsgemäß eingesetzten Struktur-Antigene des HCV sind das Kapsid-Protein Core, das Hüll-Glycoprotein E1, und das Hüll-Glycoprotein E2.

Selbstverständlich ist es nicht notwendig, zur Herstellung einer Vakzine die Struktur-Antigene von HCV vollständig zu exprimieren. Vom Experimentator können je nach Versuchsplänung und gewünschtem Resultat auch nur Teile der Struktur-Antigene zur Expression gebracht werden, insbesondere natürlich immunogene Abschnitte oder Epitope der HCV-Struktur-Antigene. Zu den immunogene Abschnitte der HCV-Struktur-Antigene. Beispiele hierfür sind die C-terminale Core-Proteinregion (AS 127-190 des HCV Polyproteins), die E1-Proteinregion der HCV-Polyprotein-AS 220-371, sowie die 27 AS umfassende hypervariable Region 1 am N-Terminus des Glykoproteins E2 (Rehermann & Chisari 2000, Curr Top Microbiol Immunol 242:299-325, Penin et al 2001, J Virol 75:5703-5710). Zu den funktionellen Teilen der Struktur-Antigene gehören bspw. die für den Zusammenbau von Virus-ähnlichen Nukleokapsid-Partikeln ausreichenden, 124 N-terminalen AS des HCV Core-Proteins (Kunkel et al. J. Virol 75:2119-2129, 2001) und die für die Heterodimerisierung der Hüllproteine E1 und E2 essentiellen Transmembran-Domänen TMD-E1 AS 350-370 bzw. TMD-E2 AS 717-740 (Op de Beeck et al. 2000, J. Biol Chem 275:31428-31437) bzw. sezernierbare Formen der Glykoproteine, die in der Lage sind, das Endoplasmatische Reticulum zu passieren. Dies wird bevorzugt dadurch erreicht, daß die Transmembran-Region von E1 bzw. E2 , durch Deletion oder anderweitige Mutationen so verändert wird, daß die Proteine nicht mehr in der Membran zurückgehalten werden (Michalak et al. 1997, J Gen Virol 78:2299-2306).

Epitope der HCV-Struktur-Antigene sind bekannt. Hierzu gehören bspw. die MHC-I-restringierten T-Zellepitope core AS 2-10, core AS 35-44, core AS 41-49, core AS 85-98, core AS 132-140, core AS167-176, core AS 178-187, core AS 181-190, E1 AS 220-227, E1 AS 233-242, E1 AS 363-371, E2 AS 401-411, E2 AS 489-496, E2 AS 621-628 oder E2 AS 725-733 (Rehermann & Chisari 2000, Curr Top Microbiol Immunol 242:299-325) Weitere Beispiele sind das E1-Epitop AS 312 - 326. Die Epitope der HCV-Struktur-Antigene können auch durch an sich bekannte Techniken bestimmt werden, die bspw. in Koziel et al 1995, J. Clin Invest 96:2311-2321 beschrieben sind.

In einer bevorzugten Ausgestaltungsform der Erfindung enthält das rekombinante MVA DNA-Sequenzen, die für E1 und E2 kodieren. Insbesondere vorteilhaft ist die Verwendung des E1-Antigens in Verbindung mit einer verkürzten Form des E2-Antigens, so daß beide Antigene co-produziert werden. "Verkürzt" heißt, daß der lipophile Anteil des E2-Glycoproteins deletiert ist. Es zeigte sich, daß in dieser Ausgestaltungsform des rekombinanten MVA nach Vakzinierung die gegen das E1-Antigen gerichteten zellulären Immunantworten, das heißt die Induktion E1-spezifischer zytotoxischer T-Zellen, deutlich verstärkt war im Vergleich zur Vakzinierung mit rekombinantem MVA, welches das E1-Antigen zusammen mit dem vollständigem E2 Protein exprimierte. Selbstverständlich ist es auch möglich, verschiedene HCV-Struktur-Antigene, insbesondere E1 und E2, nicht auf einem MVA-Vektor anzuordnen, sondern auf zumindest zwei Vektoren. Auch dies führt im Ergebnis zu einer Co-Produktion von HCV-Struktur-Antigenen.

Erfindungsgemäß wird unter Mutation eine Deletion, Basensubstitution oder eine Basenaddition verstanden. Auch chemische Modifikationen sind möglich.

Die Anordnung der HCV-Struktur-Antigene im rekombinanten MVA ist so vorzunehmen, daß eine funktionelle Verbindung der kodierenden Sequenzen mit Steuerelementen, bspw. Promotoren und Enhancern, entsteht. Geeignete DNA-Klonierungstechniken stehen dem Fachmann zur Verfügung. Es sei hier nur beispielsweise hingewiesen auf Sambrook, J. et al., 1989, Molecular Cloning: A Laboratory Manual, Cold Spring Harbor Laboratory, NY; oder Ausubel, F.M. et al., 1989, Current Protocols in Molecular Biology, John Wiley & Sons, New York, NY. Auf diese beispielhaft genannten Veröffentlichungen wird hier Bezug genommen. Insbesondere sind die kodierenden DNA-Sequenzen so anzuordnen und auszuwählen, daß eine korrekte Prozessierung der Polypeptide in den mit dem MVA-HCV-Vektorvirus infizierten Zellen erfolgt.

Die Integration der Fremd-DNA Sequenzen in das MVA-Genom erfolgt bevorzugt in solche Bereiche, die für die Replikation und Infektionsfähigkeit des MVA nicht essentiell sind. Als Integrationsorte kommen bspw. natürlich vorkommende Deletionen im MVA-Genom in Frage. Zu diesen Deletionen, die den Lebenszyklus des Virus nicht essentiell beeinflussen, gehören die Deletionen I, II, III, IV, V, und VI, bevorzugt die Deletionen II und III (Meyer, H., Sutter, G. und Mayr, A., 1991, J. Gen. Virol. 72, 1031-1038). Das so hergestellte rekombinante MVA ist infektiös, d.h. es ist in der Lage, Fremdzellen zu infizieren und die integrierte Fremd-DNA-Sequenz des HCV zu exprimieren. Derartige rekombinante Viren eignen sich als äußerst sichere Lebendvakzine zur Behandlung oder Prophylaxe von durch HCV verursachten Erkrankungen.

Das erfindungsgemäße rekombinante MVA kann, wie nachfolgend allgemein dargestellt, hergestellt werden. Eine detaillierte Beschreibung findet sich in den Ausführungsbeispielen. Das Einführen der HCV-Strukturantigene in die MVA-DNA erfolgt beispielsweise im Wege einer homologen Rekombination, wobei die zu integrierende Sequenz an beiden Enden mit solchen Sequenzen versehen wird, die mit Sequenzen rekombinierbar sind, die den natürlich vorkommenden Deletionen, beispielsweise Deletion II oder III, im MVA-Genom benachbart angeordnet sind (Antoine et al. 1998, Virology 244:365-396).

Ein derartiges DNA-Konstrukt, welches ein HCV-Strukturantigen oder funktionelle Teile oder Epitope hiervon enthält, flankiert von MVA-DNA-Sequenzen, die benachbart von den Stellen der Deletionen, beispielsweise Deletion II oder III, im MVA-Genom vorkommen, werden in mit MVA infizierte Zellen eingeführt, um eine homologe Rekombination zu ermöglichen. Das eingeführte DNA-Konstrukt kann linear oder zirkulär sein. Bevorzugt wird ein zirkuläres DNA-Molekül, insbesondere ein Plasmid, eingesetzt.

Zur Expression der für HCV-Strukturantigene codierenden DNA-Sequenz sind regulatorische Sequenzen notwendig, die eine Transkription der DNA-Sequenz ermöglichen. Derartige regulatorische Sequenzen, beispielsweise Promotoren und Enhancer, sind an sich bekannt. Hierzu gehören beispielsweise auch die Promotoren des Vaccinia 11 kDa-Gens (vergleiche EP 0 198 328), des 7,5 kDa-Gens (vergleiche EP 0 110 385) oder synthetische Vacciniavirus-spezifische Promotoren (vergleiche Sutter et al., Vaccine 1994, 12: 1032).

Das DNA-Konstrukt kann in MVA infizierte Zellen durch an sich bekannte Verfahren eingeführt werden, beispielsweise im Wege einer Transfektion durch Calciumphosphatpräzipitation (Graham et al., Virol. 52, 456-467, 1973; Wigler et al., Cell 777-785, 1979, durch Elektroporation (Neumann et al., EMBO J. 1, 841-845,1982), durch Mikroinjektion (Graessmann et al., Meth. Enzymology 101, 482-492, 1983), durch Liposomentechnologie (Straubinger et al., Methods in Enzymology 101, 512-527, 1983), durch Sphäroplastentechnologie (Schaffner, Proc. Natl. Acad. Sci. USA 77, 2163-2167, 1980) oder andere an sich bekannte Methoden. Bevorzugt wird die Calciumphophattechnik eingesetzt.

Nach Einführen des DNA-Konstrukts in die Eukaryontenzellen und nachher erfolgter Rekombination der HCV-DNA mit der viralen DNA kann das rekombinante MVA in an sich bekannter Weise isoliert werden, bevorzugt mit Hilfe eines Markergens (vergleiche Nakano et al., Proc. Natl. Acad. Sci. USA 79, 1593-1596, 1982; Franke et al., Mol. Cell. Biol. 1918-1924, 1985, Chakrabarti et al., Mol. Cell. Biol. 3403-3409, 1985, Fathi et al., Virology 97-105, 1986).

Das erfindungsgemäß hergestellte MVA kann ebenfalls ein Markergen tragen. Derartige Markergene erleichtern die Isolierung des rekombinanten Virus unter Zuhilfenahme an sich bekannter Techniken. Derartige Markergene sind an sich bekannt, und hierzu gehören Gene, die für Proteine wie β-Galactosidase, Neomycin, Alkoholdehydrogenase, Luciferase, Puromycin, Hypoxanthin-Phosphoribosyltransferase (HPRT), Hygromycin, sezernierte alkalische Phosphatase oder Grün- und Blau-Fluoreszenzproteine.

Selbstverständlich sind auch andere Methoden zur Herstellung des rekombinanten Virus der vorliegenden Erfindung möglich, beispielsweise die im nachfolgenden Ausführungsbeispiel näher beschriebene Klonierungsmethode.

### Wachstum und Reinigung der Viren

### Wachstum des MVA-Virus

Das MVA-Virus ist ein stark abgeschwächtes Vaccinia-Virus, das vom Vaccinia-Virusstamm Ankara (CVA) durch serielle Langzeit-Passagen auf primären Hühnerembryo-Fibroblasten-(CEF)-Kulturen hergeleitet ist. Für eine allgemeine Übersicht über die Geschichte der Produktion, der Eigenschaften und der Verwendung des MVA-Stammes kann auf die Zusammenfassung Bezug genommen werden, die von Mayr et al. in Infection 3, 6-14 [1975] veröffentlicht wurde. Aufgrund der Abschwächung in CEF repliziert das MVA-Virus zu hohen Titern in dieser Vogel-Wirtszelle. In Säugerzellen ist das Wachstum von MVA jedoch stark gehemmt, und eine typische Plaquebildung durch das Virus ist nicht nachweisbar. Das MVA-Virus wurde daher auf CEF-Zellen gezüchtet. Zur Herstellung von CEF-Zellen wurden 11 Tage alte Embryos aus inkubierten Hühnereiern isoliert, die Extremitäten wurden entfernt, und die Embryos wurden zerkleinert und in einer Lösung, die aus 0,25% Trypsin bestand, 20 min lang bei 37°C dissoziiert. Die resultierende Zellsuspension wurde filtriert, und die Zellen wurden durch Zentrifugation bei 2000 U/min in einer Sorvall RC-3B-Zentrifuge 5 min bei Raumtemperatur sedimentiert, in 10 Volumina Medium A (MEM Eagle, bspw. erhältlich von Life Technologies GmbH Eggenstein, Deutschland) resuspendiert und erneut 5 min durch Zentrifugation bei 2000 U/min in einer Sorvall RC-3B Zentrifuge bei Raumtemperatur sedimentiert. Das Zellpellet wurde in Medium A, das 10 % fötales Kälberserum, (FCS), Penicillin (100 Units/ml), Streptomycin (100 mg/ml) und 2 mM Glutamin wieder aufgeschlämmt, so dass eine Zellsuspension erhalten wurde, die 500 000 Zellen/ml enthielt. Derart erhaltene CEF-Zellen wurden auf Zellkulturschalen verteilt. Diese wurden je nach gewünschter Zelldichte in Medium A in einem CO₂-Inkubator 1-2 Tage bei 37°C wachsen gelassen, und wurden entweder direkt oder nach einer weiteren Zellpassage für die Infektion verwendet. Eine eingehende Beschreibung der Herstellung von Primärkulturen befindet sich in dem Buch von R.I. Freshney, "Culture of animal cell", Alan R. Liss Verlag, New York [1983] Kapitel 11, S. 99ff.

MVA-Viren wurden wie folgt zur Infektion verwendet. Die CEF-Zellen wurden in 175 cm² Zellkulturflaschen gezüchtet. Bei 90 bis 100%iger Konfluenz wurde das Medium entfernt, und die Zellen wurden für eine Stunde mit einer MVA-Virussuspension (0,01 infektiöse Einheiten (IU) pro Zelle, 0,02 ml/cm²) in Medium A inkubiert. Dann wurde mehr Medium A dazugegeben (0,2 ml/cm²), und die Flaschen wurden 2 bis 3 Tage bei 37°C inkubiert (bis etwa 90% der Zellen cytopathogene Wirkung zeigten). Rohe Virusstammpräparate wurden hergestellt, indem die Zell-Monolayer in das Medium geschabt und das Zellmaterial durch Zentrifugation bei 3000 U/min in einer Sorvall RC-3B Zentrifuge 5 min bei 4°C pelletiert wurden. Das rohe Viruspräparat wurde vor der weiteren Verarbeitung (z.B. Virusreinigung) bei -20°C aufbewahrt.

### Reinigung der Viren

Die Reinigungsschritte, die unternommen wurden, um ein Viruspräparat zu erhalten, das so rein wie möglich war und keine für die Wirtszelle spezifischen Komponenten enthielt, ähnelten denjenigen, die von Joklik (Virology 18, 9-18 [1962]) beschrieben wurden. Rohe Virusstammpräparate, die bei -20°C aufbewahrt worden waren, wurden aufgetaut und einmal in PBS suspendiert (das 10 bis 20fache des Sedimentvolumens), und die Suspension wurde wie oben zentrifugiert. Das neue Sediment wurde im 10fachen Volumen Tris-Puffer 1 (10 mM Tris-HCl, pH-Wert 9,0) suspendiert, und die Suspension wurde kurz mit Ultraschall behandelt (Labsonic, L.B. Braun Biotech International, Melsungen, Deutschland; 2 x 10 sec bei 60 Watt und Raumtemperatur), um die Zelltrümmer weiter zu zerkleinern und um die Viruspartikel aus dem zellulären Material freizusetzen. Die Zellkerne und die größeren Zelltrümmer wurden in der nachfolgenden kurzen Zentrifugation der Suspension (Sorvall GSA-Rotor-erhältlich von DuPont Co., D-6353 Bad Nauheim, Deutschland, 3 min bei 3000 U/min und 10°C) entfernt. Das Sediment wurde noch einmal in Tris Puffer 1 suspendiert, mit Ultraschall behandelt und wie vorstehend beschrieben zentrifugiert. Die gesammelten Überstände, die frei von Viruspartikeln sind, wurden vereinigt und über ein Kissen aus 10 ml 36%iger Saccharose in 10 mM Tris-HCl, pH-Wert 9,0 geschichtet und in einem Beckman SW 27/28-Rotor 80 min bei 13500 U/min bei 4°C zentrifugiert. Der Überstand wurde verworfen, und das Sediment, das die Viruspartikel enthielt, wurde in 10 ml 1 mM Tris-HCl, pH-Wert 9,0 aufgenommen, durch kurze Behandlung mit Ultraschall (2 x 10 sec bei Raumtemperatur, Gerät wie vorstehend beschrieben) homogenisiert und auf einen 20-40% Saccharosegradienten (Saccharose in 1 mM Tris-HCl, pH-Wert 9,0) zur weiteren Reinigung aufgetragen. Der Gradient wurde 50 min in einem Beckman SW41 Rotor bei 13000 U/min und 4°C zentrifugiert. Nach der Zentrifugation wurden getrennte Banden, die Viruspartikel enthielten, durch Pipettieren geerntet, nachdem das Volumen über der Bande abgesaugt wurde. Die erhaltene Saccharoselösung wurde mit drei Volumina PBS verdünnt, und die Viruspartikel wurden wiederum durch Zentrifugation (Beckman SW 27/28, 60 min bei 13500 U/min, 4°C) sedimentiert. Das Pellet, das nun überwiegend aus reinen Viruspartikeln bestand, wurde in PBS resuspendiert und auf Virus-Konzentrationen äquilibriert, die im Durchschnitt 1 bis 5 x 10⁹ IU/ml entsprachen. Die gereinigte Virus-Stammlösung wurde bei -80°C aufbewahrt und entweder direkt verwendet oder für die nachfolgenden Experimente mit PBS verdünnt.

Das modifizierte Vaccinia-Virus Ankara (MVA), ein stark abgeschwächter Vaccinia-Virusstamm mit eingeschränktem Wirtsspektrum, kann sich in vom Menschen stammenden Zellen und den meisten anderen untersuchten Säugerzelllinien nicht vermehren. Da jedoch die virale Genexpression in nichtpermissiven Zellen nicht beeinträchtigt ist, kann das erfindungsgemäße rekombinante MVA als ungewöhnlich sicherer und effizienter Expressionsvektor und rekombinante Vakzine verwendet werden.

Bei einer erfindungsgemäßen Ausführungsform wurden somit rekombinante MVA-Viren konstruiert, die die Expression der HCV-Strukturantigene unter der Kontrolle des Vaccinia-Virus-Early/Late-Promotors P7.5 ermöglichen.

Das rekombinante MVA-Virus, welches ein HCV-Strukturantigen exprimiert, kann z.B. zur Immunisierung für die Immuntherapie bei Krebspatienten verwendet werden.

Darüber hinaus kann das rekombinante MVA-Virus, welches HCV-Strukturantigene exprimiert, in Verbindung mit antigenpräsentierenden Zellen, bspw. Dendritenzellen, Makrophagen und B-Zellen, zur Immunisierung von Menschen für die Immuntherapie verwendet werden.

Das rekombinante MVA-Virus kann überdies in Verbindung mit antigenpräsentierenden Zellen, bspw. Dendritenzellen, ex vivo zur Erzeugung von Immuneffektorzellen verwendet werden, welche an Menschen zur Immuntherapie applizierbar sind.

Das rekombinante MVA-Virus kann darüber hinaus zur Herstellung von rekombinanten HCV-Strukturproteinen verwendet werden.

Die erfindungsgemäßen MVA-Vaccinia-Viren werden zur Herstellung von Vakzinen oder Therapeutika in eine physiologisch verträglich Form umgewandelt. Hier kann man auf die Erfahrung bei der Herstellung von MVA-Vakzinen zurückgreifen, die zur Impfung gegen Pocken verwendet werden (wie beschrieben von Stickl, H., et al. [1974] Dtsch. med. Wschr. 99, 2386-2392). Gewöhnlich werden etwa 10⁶ bis 10⁸ rekombinante MVA-Partikel in 100 ml phosphatgepufferter Salzlösung (PBS) in Gegenwart von 2% Pepton und 1% menschlichem Albumin in einer Ampulle, vorzugsweise einer Glasampulle, gefriergetrocknet. Das Lyophilisat kann Streckmittel (wie Mannitol, Dextran, Zucker, Glycin, Lactose oder Polyvinylpyrrolidon) oder andere Hilfsmittel (wie Antioxidantien, Stabilisatoren, usw.) enthalten, die sich zur parenteralen Verabreichung eignen. Die Glasampulle wird dann verschlossen, und kann dann, vorzugsweise bei Temperaturen unter -20°C, mehrere Monate lang aufbewahrt werden.

Zur Impfung oder zur Therapie kann das Lyophilisat in 0,1 bis 0,5 ml wässriger Lösung, vorzugsweise in physiologischer Kochsalzlösung, gelöst werden, und entweder parenteral, bspw. durch intramuskuläre Impfung, oder lokal, bspw. durch intradermale Impfung oder durch Impfung in einen Tumor oder an die Stelle eines Tumors, verabreicht werden. Erfindungsgemäße Vakzine oder Therapeutika werden vorzugsweise intramuskulär injiziert (Mayr, A. et al. [1978] Zbl. Bakt. Hyg., I. Abt. Orig. B 167, 375-390). Der Verabreichungsweg, die Dosis und die Anzahl der Verabreichungen kann durch den Fachmann auf bekannte Weise optimiert werden. Geeignetenfalls ist es angebracht, den Impfstoff mehrmals für einen längeren Zeitraum zu verabreichen, damit geeignete Immunantworten gegen das fremde Antigen erhalten werden.

Die Erfindung umfaßt auch Eukaryontenzellen, die mit den erfindungsgemäßen rekombinanten MVA-Viren infiziert sind. Hierzu gehören bspw. Hühnerembryo-Fibroblastenzellen, Babyhamster-Nierenzellen, bevorzugt BHK-21-Zellen, oder Antigen-präsentierende Zellen wie z. B. dendritische Zellen, Makrophagen oder B-Lymphozyten.

Die rekombinanten MVA-Viren werden, wie oben näher beschrieben, zur Herstellung einer Vakzine eingesetzt, die zur Therapie und Prophylaxe von HCV-Infektionen und hierdurch verursachter Erkrankungen, insbesondere chronischer Lebererkrankungen und Lebertumoren, einsetzbar ist. Mit Hilfe des rekombinanten Virus können natürlich auch die rekombinanten HCV-Strukturproteine produziert und nach Isolierung und Aufreinigung in reiner Form gewonnen werden. Unter rekombinanten HCV-Proteinen sind erfindungsgemäß HCV-Strukturantigene zu verstehen, wobei dieser Ausdruck auch funktionelle Teile der HCV-Strukturantigene einschließt oder Epitope, insbesondere Oberflächenepitope der HCV-Strukturantigene.

Die Erfindung umfaßt auch DNA oder RNA des rekombinanten MVA.

Nachfolgend wird die Erfindung anhand von Ausführungsbeispielen näher beschrieben. Die Erfindung ist jedoch nicht auf diese besondere Ausgestaltung beschränkt sondern kann im Rahmen der Beschreibung in Verbindung mit den beiliegenden Patentansprüchen abgewandelt werden.

Die beiliegenden Figuren dienen ebenfalls dem näheren Verständnis der Erfindung, insbesondere der Ausführungsbeispiele. Die Figuren zeigen:

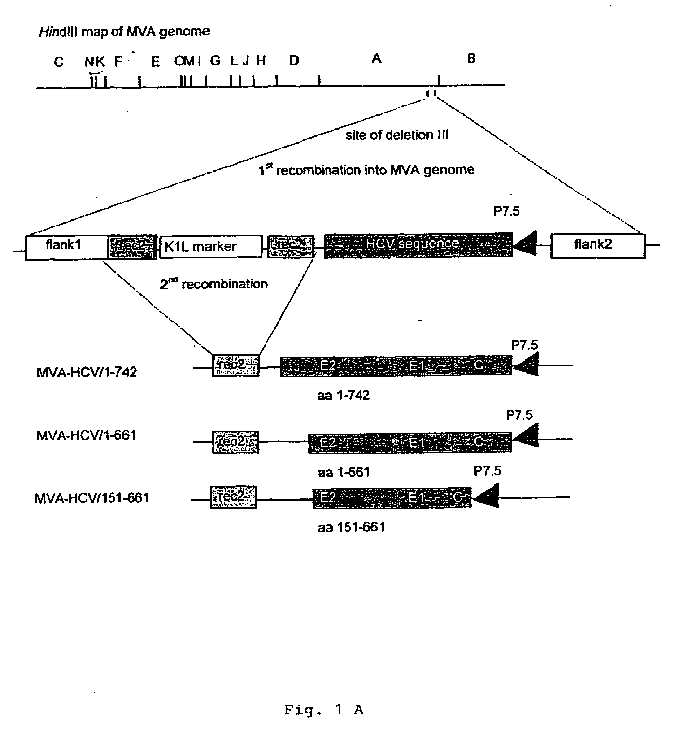

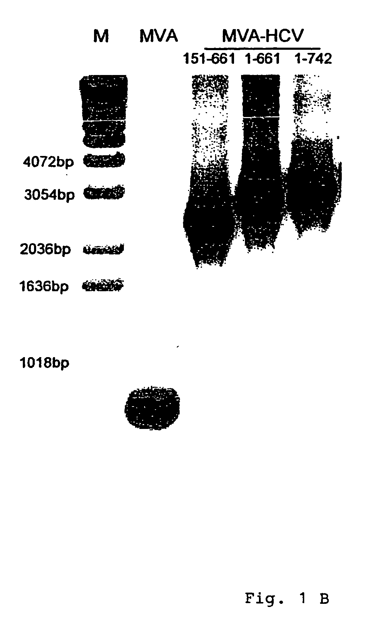

**Fig. 1** **Konstruktion und genomische Struktur rekombinanter MVAenthaltender Gensequenzen für HCV-Strukturproteine.** (A) Eine schematische Karte des MVA-Genoms für die Restriktionsendonuklease HindIII. Die HCV-codierenden Sequenzen standen unter der transkriptionellen Kontrolle des Vaccinia-Virus-Early/Late Promoters P7.5 und wurden durch homologe Rekombination an der Stelle der Deletion III innerhalb des MVA-Genoms inseriert. Die Flanke 1 und Flanke 2 betreffen MVA-DNA-Sequenzen, die sich neben der Deletionsstelle III befinden und dienen dazu, die Rekombination in das MVA-Genom zu dirigieren. Rec2 zeigt die Positionen der 283 bp repetitiven MVA-DNA-Sequenzen, die dem rechten Ende von Flanke 1 entsprechen und die Entfernung des K1L-selektierbaren Markers aus dem Genom der fertigen rekombinanten Viren durch homologe Rekombination ermöglichen. Die Darstellungen der Genomstrukturen der rekombinanten MVA-HCV-151-661, MVA-HCV-1-661 und MVA-HCV-1-742 sind unten gezeigt. (B) PCR-Analyse viraler DNA zur Überwachung von Gensequenzen, die an der Deletionsstelle III inseriert sind. Genomische DNA von Wildtyp-MVA und rekombinantem MVA-HCV-151-661, MVA-HCV-1-661 und MVA-HCV-1-742 dienten als Matrizen-DNA zur Amplifikation charakteristischer DNA-Fragmente, welche durch Agarosegelelektrophorese aufgetrennt wurden. Die 1-kB-Leiter (Gibco) wurde als Marker (M) für das Molekulargewicht in Basenpaaren (bp) angegeben.

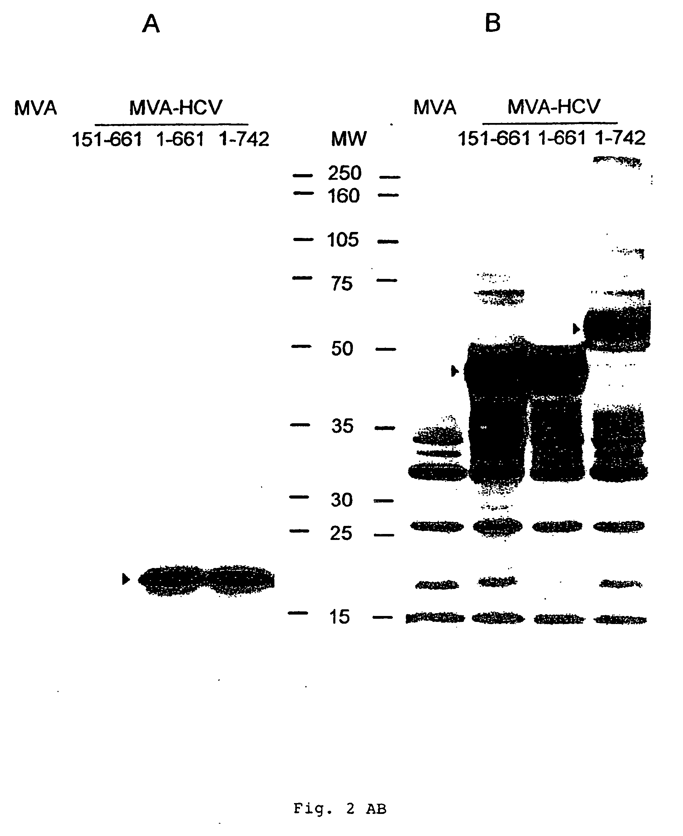

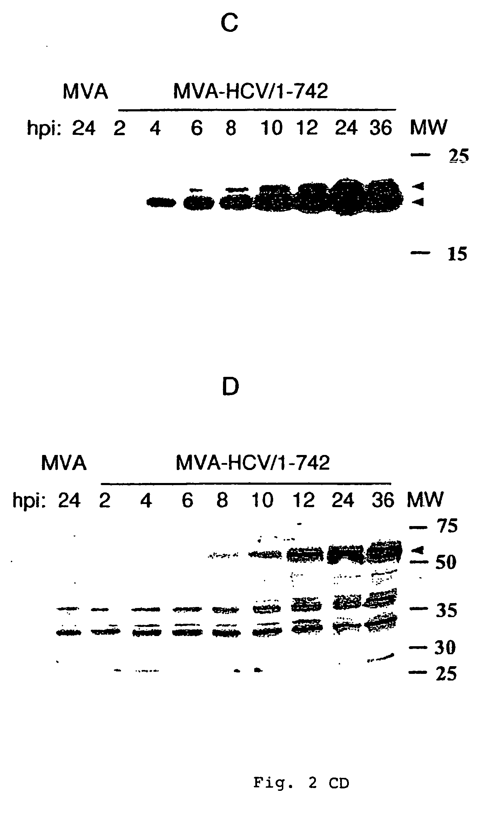

**Fig. 2****. Synthese von HCV-Core- und E2-Proteinen durch rekombinante MVA.** BHK-21-Zellen wurden mit 10 IU/Zelle MVA oder MVA-HCV infiziert und nach 24 Std. (A, B) oder zu den angegebenen Zeitpunkten (C, D) nach der Infektion geerntet. Proteine aus Zelllysaten wurden durch SDS-10% PAGE getrennt und durch Western-Blot mittels monoklonalen Maus-anti-HCV-Core (A,C)- oder E2-spezifischen polyklonalen Kaninchen-Antikörpern (B, D) analysiert. HCV-spezifische Proteinbanden sind durch Pfeilspitzen markiert. Die Positionen und Molekülmassen (in kDa) der Proteinstandards sind in den Spuren MW gezeigt.

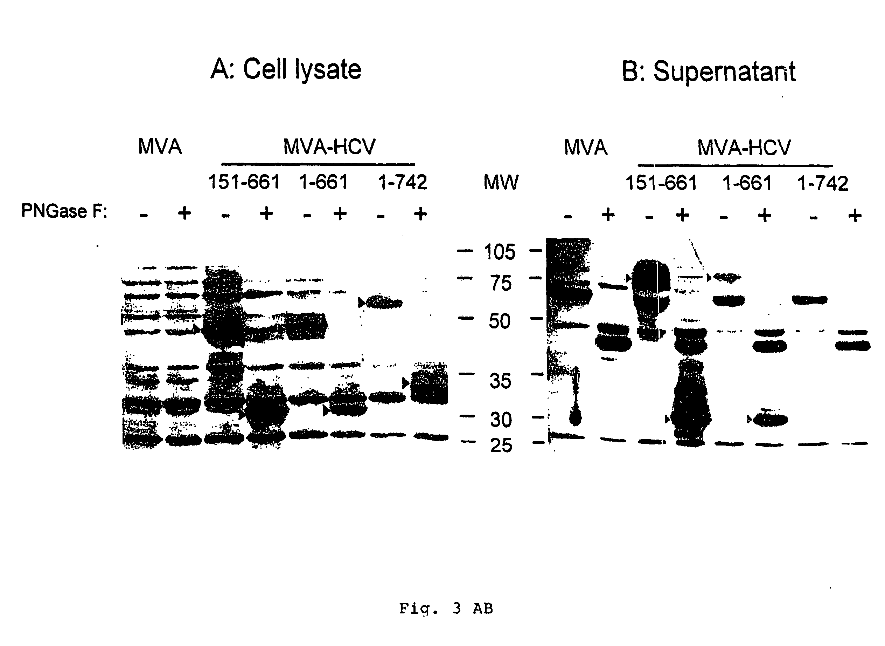

**Fig. 3** **Glycosylierung der durch rekombinante MVA hergestellten zellassoziierten und sezernierten E2-Proteine.** Gesamt-Zellproteine (A) oder Proteinpräzipitate von Überständen (B) aus MVA-infizierten BHK-21-Zellen wurden in SDS/β-Mercaptoethanol denaturiert, in Gegenwart (+) oder Abwesenheit (-) von Endoglycosidase PNGase F inkubiert, auf SDS-10%-PAGE getrennt und durch Western-Blot mit polyklonalem Kaninchen-anti-E2-Serum analysiert. HCV-E2-spezifische Proteinbanden sind durch Pfeilspitzen markiert. Die Zahlen in der Spur MW betreffen die Positionen und Molekulargewichte (kDa) der Proteinstandards.

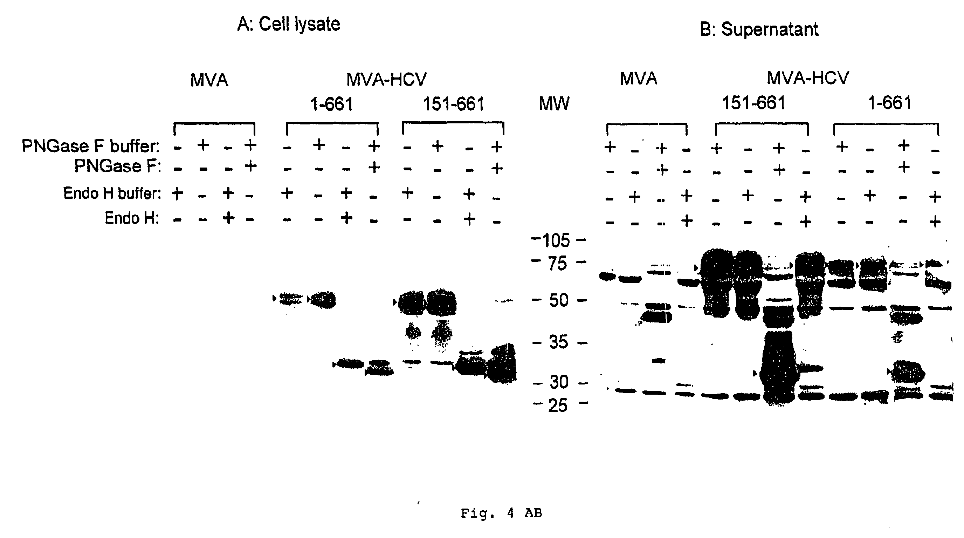

**Fig. 4** **Analyse der EndoH-Empfindlichkeit der durch rekombinante MVA hergestellten zellassoziierten und sezernierten E2-Proteine.**
   BHK-21-Zellen wurden 24 Std. mit rekombinantem MVA-HCV/151-661 oder MVA-HCV/1-661 oder nicht-rekombinantem MVA bei einer MOI von 10 infiziert. Gesamt-Zellproteine (A) oder Proteinpräzipitate der Überstände (B) aus infizierten Kulturen wurden in SDS/β-Mercaptoethanol denaturiert, in Gegenwart (+) oder Abwesenheit (-) von PNGase F oder EndoH inkubiert, auf SDS-10%-PAGE getrennt und durch Western-Blot mit polyklonalem Kaninchen-anti-E2-Serum analysiert. HCV-E2-spezifische Proteinbanden sind durch Pfeilspitzen markiert. Die Zahlen in der Spur MW betreffen die Positionen und Molekulargewichte (kDa) der Proteinstandards.

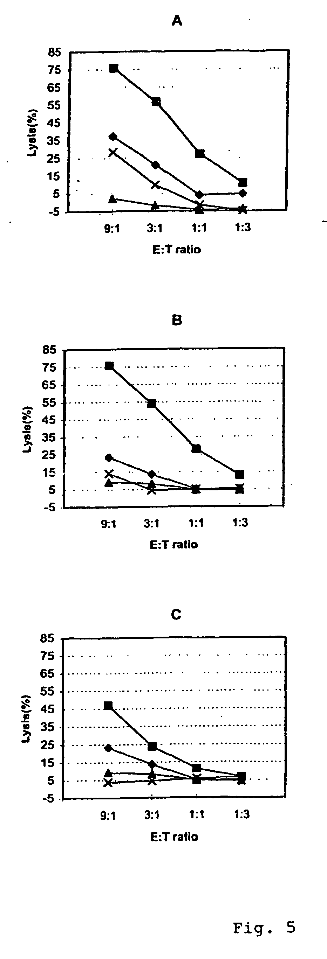

**Fig. 5** **Spezifische Lyse von E1-Peptid-gepulsten Zielzellen durch CTL aus MVA-HCV-geimpften BALB/c-Mäusen.** Splenocyten wurden aus Mäusen erhalten, die mit rekombinantem MVA-HCV/151-661 (A), MVA-HCV/1-661 (B) oder MVA-HCV/1-742 (C) geimpft worden waren, und mit E1-Peptid AS 312-326 in vitro restimuliert wurden. Diese Effektoren wurden auf Cytotoxizität im angegebenen E:T-Verhältnis in einem 4stündigen ⁵¹Cr-Freisetzungstest gegen P815-Target untersucht, das mit E1-Peptid AS 312-326 (■) oder Flu-Peptid (▲) gepulst worden war. Splenocyten aus Mäusen, die mit nicht-rekombinanten MVA geimpft worden waren, dienten als Kontrollen und wurden auf P815-Targets untersucht, die mit E1-Peptid AS 312-326 (◆) oder Flu-Peptid (X) inkubiert worden waren.

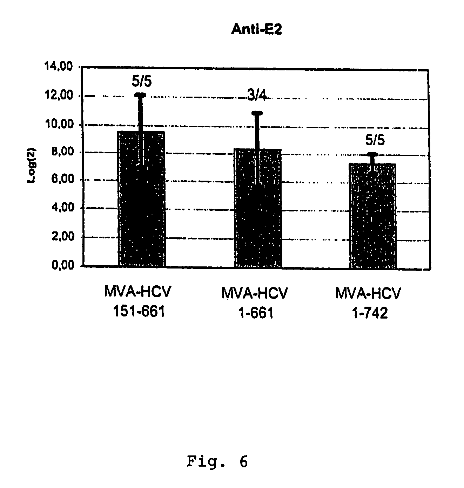

**Fig. 6** **E2-spezifische Antikörper-Antwort in BALB/c-Mäusen, die mit rekombinantem MVA geimpft worden waren.** Zweifache Verdünnungsreihen von Seren aus BALB/c-Mäusen, die dreimal mit 10⁸ IU rekombinantem oder Wildtyp-MVA immunisiert worden waren, wurden mittels ELISA auf E2-spezifische Antikörper untersucht. Die Log(2)-Werte der normalisierten mittleren anti-E2-Titer sämtlicher serokonvertierender Tiere sind gezeigt. Die Standardabweichungen sind durch Balken ausgedrückt. Die Zahlen oben zeigen die Verhältnisse der serokonvertierenden Tiere zu den insgesamt analysierten Tieren.

### Materialien und Methoden

### Zelllinien und Viren

Primäre Hühnerembryo-Fibroblasten (CEF), Baby-Hamster-Nieren-BHK-21- (ATCC CCL-10), Kaninchen-Nieren-RK-13- (ATCC CCL-37) und Maus-P815 (H-2d, ATCC TIB64)-Zellen wurden in minimalem essentiellem Medium (MEM) oder RPMI-1640-Medium, das mit 10% fötalem Kälberserum (FCS) supplementiert war, gezüchtet. Die Zellen wurden in einer Feuchtluft-5%-CO₂-Atmosphäre bei 37°C gehalten.

Vaccinia-Virus MVA (kloniertes Isolat F6, 45, 60) wurde routinemäßig vermehrt und sein Titer durch eine Vaccinia-Virus-spezifische Immunfärbung in CEF ermittelt, um die infektiösen Einheiten (infectious units, IU)/ml zu bestimmen. Virus,aus dem 582. CEF-Durchlauf wurde für diese Untersuchung verwendet. Rekombinantes Virus MVA-LZ, das das *E. coli* lacZ-Reportergen codiert (60), wurde im Plaque-Reduktionsassay verwendet, um Vaccinia-Virus-MVA-spezifische neutralisierende Antikörper zu bestimmen.

### Plasmidkonstrukte

Zur Konstruktion von MVA-Vektorplasmiden, die HCV-Strukturgene enthalten, wurden DNA-Fragmente aus klonierter HCV-cDNA hergestellt, die ursprünglich von Y. Wang (Universität Peking, GeneBank Zugangs-Nr. D10934, 63, 38) zur Verfügung gestellt wurde. Ein DNA-Sement, das die codierende Sequenz für die HCV-Aminosäuren (AS) 1 bis 742 enthielt, wurde durch EcoRI- und HindIII-Spaltung von pUC18/CE1E2 erhalten, mit Klenow-Polymerase zur Erzeugung stumpfer Enden behandelt und in die einzige PmeI-Stelle von pIIIdHR-P7.5 kloniert, so dass pIIIdHR-P7.5-HCV(1-742) erhalten wurde.

Zur Gewinnung der MVA-Vektorplasmide pIIIdHR-P7.5-HCV(1-661) und pIIIdHR-P7.5-HCV(151-661) wurden DNA-Fragmente, die die HCV-AS 1-661 oder AS 151-651 codieren, mittels Polymerasekettenreaktion (PCR) aus der gleichen HCV-cDNA-Matrize amplifiziert, wobei folgende Oligonukleotide verwendet wurden: HCV-5'-1 5'-CAT GGG AAT TCC CAT GAG-3', HCV-5'-151 5'-GGC GCT GCG AAT TCC ATG GCG CAT GGC GTC CGG-3' und HCV-3'-661 5'-GGG GGG GAA TTC TCA CTC TGA TCT ATC CCT GTC-3' (EcoRI-Stellen sind unterstrichen). Die PCR-Produkte wurden mit EcoRI gespalten, mit Klenow stumpfendig gemacht und in die PmeI-Stelle von pIIIdHR-P7.5 kloniert.

### Erzeugung rekombinanter Viren

Monolayer nahezu konfluenter BHK-21-Zellen in Gewebekulturplatten mit sechs Vertiefungen (Corning, Corning, NY, USA) wurden mit MVA bei einer Multiplizität von 0,01 infektiösen Einheiten pro Zelle infiziert und 90 Min nach der Infektion mit 1,5 µg Plasmid-DNA pro Vertiefung mittels FuGENE 6-Transfektions-Reagenz (Roche Molecular Biochemicals, Mannheim, Deutschland) gemäß den Herstellerangaben transfiziert. 24 Std. nach der Infektion wurden die Zellen geerntet und wie zur Isolation von rMVA beschrieben durch Selektion auf transiente Erweiterung des Wirtszellspektrums verarbeitet (58). Rekombinante Viren wurden nach Bildung typischer Infektionsherde in RK-13-Zellmonolayern gesichtet. Die RK-13 Zellen unterstützen das produktive Wachstum parentaler MVA nicht. Nach drei Vermehrungszyklen der Plaquereinigung in RK-13-Zellen wurden rMVA durch BHK-21-Zellen geschickt, um das selektierbare Markergen K1L zu entfernen. Virus-Ausgangsmaterialien wurden in BHK-21-Zellen hergestellt, durch eine Zweischritt-Ultrazentrifugation durch ein Saccharosekissen gereinigt, der Titer auf CEF-Zellen mittels Vaccinia-Virus-spezifischer Immunfärbung bestimmt, aliquotiert und bei -80°C aufbewahrt.

### Analyse rekombinanter Proteine

Monolayer von BHK-21-Zellen wurden mit 10 infektiösen rMVA-Einheiten pro Zelle infiziert. Nach 24 Std. wurden die infizierten Zellen geerntet, durch Zentrifugation aufgefangen und in SDS-Gelbeladungspuffer (50 mM Tris-Cl, pH-Wert 6,8, 2% SDS, 0,04% Bromphenolblau, 84 mM β-Mercaptoethanol, 20% Glycerin) lysiert. Gesamt-Zellproteine wurden in einem SDS-Polyacrylamidgel elektrophoretisch aufgetrennt und auf Nitrocellulose elektrogeblottet. Die Blot-Membranen wurden 1 Std. in phosphatgepuffertem Salinepuffer (PBS) mit 2% (Gew./Vol.) Rinderserumalbumin (BSA) und 0,05% (Vol./Vol.) Tween 20 blockiert, dann 4 Std. mit polyklonalen Kaninchen-anti-HCV-E2-Antikörpern (Serum RE2116, 1:5000), polyklonalen Kaninchen-anti-HCV-Antikörpern (Antigenix Amerika Inc./Bio-Trade, Wien, Österreich, 1:10000) oder monoklonalen Maus-anti-HCV-Core-Antikörpern C1 (1:5000, freundlicherweise von Ramsey C. Cheung, Stanford University School of Medicine, Stanford, CA, USA zur Verfügung gestellt), verdünnt im gleichen PBS-Puffer, inkubiert. Nach Waschen mit 0,05% Tween 20 in PBS, wurden die Blots 1 Std. mit anti-Kaninchen- oder anti-Maus-IgG-Antikörper, konjugiert an Meerrettich-Peroxidase (Dianova, Hamburg, Deutschland), und 10000-fach in Blockierungspuffer verdünnt, inkubiert, erneut gewaschen und mittels Lumi-Light Westerri-Blotting-Substrat (Roche Molecular Biochemicals, Mannheim, Deutschland) entwickelt. Zur Überwachung sezernierter rekombinanter HCV-Proteine wurden zellfreie Überstände aus MVA-infizierten Kulturen, die in serumfreiem optiMEM (Gibco BRL) gezüchtet wurden, 24 Std. nach der Infektion gesammelt. Proteine aus den Überständen wurden mit einem gleichen Volumen eiskaltem Ethanol gefällt, durch einstündige Zentrifugation bei 10000 x g/4°C gewonnen, in PBS resuspendiert und einer Western-Blot-Analyse, wie beschrieben, unterworfen.

Die Endoglycosidasen EndoH und PNGaseF (New England Biolabs, Frankfurt am Main, Deutschland) wurden zur Deglykosylierung rekombinanter Proteine verwendet. Die aus infizierten Zellen oder aus Kulturüberständen gefällten Gesamtproteine wurden in 0,5% SDS, 1% β-Mercaptoethanol, 10 Min. bei 100°C denaturiert, 2 Std. bei 37°C mit EndoH oder PNGase F, wie vom Hersteller beschrieben, gespalten und durch Western-Blot analysiert.

### Tierimmunisierung und Gewinnung von Serumproben

Gruppen von fünf BALB/c-Mäusen (6 bis 8 Wochen alt, erhalten von Charles River, Sulzfeld, Deutschland) wurden intraperitoneal mit 1 x 10⁸ infektiösen MVA- oder rMVA-Einheiten (in 0,5 ml sterilem PBS) pro Tier an den Tagen 0, 38 und 81 immunisiert. Die Mäuse wurden unter Anästhesie durch den Retroorbital-Plexus an den Tagen -7, 21, 48, 103 und 158 geblutet. Das Blut wurde in einem Mikrozentrifugenröhrchen gesammelt, 4 Std. bei Raumtemperatur inkubiert und 10 Min. bei 2700 x g/4°C zentrifugiert. Die erhaltenen Seren wurden bei -20°C aufbewahrt.

### Antikörpermessung durch ELISA

Enzym-Immunosorbentassays (ELISA) wurden zur Bestimmung des Vorhandenseins von Antikörpern gegen HCV-Kern- und E2-Antigene in Serumproben verwendet. Die zur Beschichtung von Flachbodenplatten mit 96 Vertiefungen (MaxiSorp Surface, Nunc, Wiesbaden, Deutschland) verwendeten Antigene bei einer Konzentration von 1 µg/ml waren HCV-Kern-Protein (AS 2 bis 192, Bio-Trade, Wien, Austria) oder in *E. coli* produziertes HCV E2 (AS 450 bis 565). Die Antigene wurden in PBS mit 0,02% Natriumazid suspendiert, mit 50 µl/Vertiefung plattiert und über Nacht bei 4°C inkubiert. Anschließend wurde der Inhalt jeder Vertiefung entfernt und dreimal mit PBS plus 0,05% Tween 20 (PBS-T) gewaschen. Blockierungspuffer (1% BSA in PBS-T) wurde bei 200 µl/Vertiefung dazu gegeben, und die Platten wurden 1 Std. bei 37°C inkubiert. Die Platten wurden mit PBS-T gewaschen, und zweifache serielle Verdünnungen der Serumproben in Blockierungspuffer wurden in einem Volumen von 100 µl/Vertiefung zugegeben und 2 Std. bei 37°C inkubiert. Nach drei Waschschritten mit PBS-T, wurde alkalische Phosphatase-konjugiertes Ziege-anti-Maus-Immunglobulin G (Dianova, Hamburg, Deutschland, 1000fach in Blockierungspuffer verdünnt) zugegeben und 1 Std. bei 37°C inkubiert. Nach achtmaligem Waschen der Platten mit PBS-T wurden die Vertiefungen mit para-Nitrophenylphosphat-Substrat (Sigma, Deisenhofen, Deutschland) entwickelt. Nach 30 Min. Inkubation bei Raumtemperatur wurde die Reaktion durch Zugabe von 0,5 M NaOH gestoppt, und die Extinktion wurde bei 405 nm mit einem Mikroplatten-Lesegerät (Model 550, Bio-Rad Laboratories, München, Deutschland) gemessen. Die Antikörper-Titer wurden als zweifache serielle Verdünnung berechnet, die eine optische Dichte (OD) ergab, die doppelt so groß wie der Cutoff war. Der Cutoff-Wert wurde bestimmt als die mittlere OD von Seren aus Kontrollmäusen, die mit nicht-rekombinantem MVA geimpft worden waren.

### Analyse von MVA-spezifischen Antikörper-Antworten

Vaccinia-Virus-MVA-spezifische Antikörper wurden durch einen Plaque-Reduktionstest mittels rekombinantem MVA-LZ analysiert. Zweifache serielle Verdünnungen von Seren aus immunisierten Mäusen wurden mit 200 infektiösen MVA-LZ-Einheiten in einem Gesamtvolumen von 200 µl PBS umgesetzt und 2 Std. bei 37°C inkubiert. Anschließend wurden konfluente BHK-21-Monolayer im Doppelansatz infiziert, und Herde virusinfizierter Zellen wurden 48 Std. nach dem Animpfen durch Färben mit 5-Brom-4-chlor-3-indolyl-β-galactopyranosid-Substrat (X-Gal, Roche Molecular Biochemicals, Mannheim, Deutschland) wie zuvor beschrieben sichtbar gemacht (16). Die blau gefärbten Herde wurden gezählt, und die bei jedem Serum erhaltene Anzahl wurde mit Kontrollen mit Maus-Präimmunseren oder ohne Maus-Serum verglichen. Die Antikörper wurden als zweifache serielle Verdünnung berechnet, die eine 50%ige Reduktion der Herd-Anzahl ergab.

### Splenozyten-Kulturen und Cytotoxizitäts-Tests

Die Verfahren erfolgten im Wesentlichen wie zuvor beschrieben (5). Splenozyten, erhalten 12 Wochen nach der letzten Virus-Beimpfung der Mäuse, wurden als In-vitro-Responder-Zellen verwendet. 5 x 10⁶ Zellen wurden in Gegenwart von 5 µg/ml E1-Peptid (HCV AS 312-326) in RPMI1640-Medium gezüchtet, das mit 10% FCS, 25 µM 2-Mercaptoethanol, 1 mM Natriumpyruvat, und 2 mM L-Glutamin supplementiert war. Am zweiten Tag wurde 10% TCGF zu den Kulturen gegeben. Nach 7 Tagen wurden die Effektorzellen geerntet und auf spezifische Cytotoxizität untersucht, wobei ein ⁵¹Cr-Freisetzungs-Test verwendet wurde. 1 x 10⁶ P815- oder T2-Targetzellen wurden mit 75 µCi Na⁵¹CrO₄ in 200 µl RPMI/10% FCS 1 Std. bei 37°C inkubiert. Die Zellen wurden dreimal mit RPMI/10%FCS gewaschen und dreimal mit 5 µg/ml E1-Peptid (HCV AS 312-326) oder 1 µg/ml A/PR/8/34-Influenza-Virus-Matrixprotein-M1-Peptid 30 min bei 37°C inkubiert. Responder- und Target-Zellen wurden in verschiedenen Verhältnissen in Platten mit 96 Vertiefungen 5 Std. bei 37°C cokultiviert. 30 µl Überstand wurde geerntet, um die Menge an freigesetztem ⁵¹Cr zu bestimmen. Sämtliche Proben wurden in einem TopCount NXT (Packard, Downers Grove, IL) gezählt, und der Prozentsatz an spezifischer Freisetzung wurde berechnet als: [(experimentelle Freisetzung - spontane Freisetzung)/(Gesamtfreisetzung - spontane Freisetzung)] x 100. Die Freisetzung wurde in Zählimpulsen pro min gemessen. Die Tests erfolgten im Doppelansatz. Zur Restimulation wurden 5 x 10⁶, aus nicht immunisierten BALB/c Mäusen stammende, bestrahlte Splenocyten (3000 rad), die 2 Std. mit 10 µg/ml E1-Peptid (HCV AS 312-326) inkubiert worden waren, zu 1 x 10⁶ Effektorzellen zugegeben und 7 Tage in RPMI1640-Medium, supplementiert mit 10% FCS, 25 µM 2-Mercaptoethanol, 1 mM Natriumpyruvat, 2 mM L-Glutamin und 10% TCGF, gezüchtet.

### Ergebnisse

### Design und Isolation von rMVA zur Expression von HCV-C-E1-E2-Genen

Vorhergehende Untersuchungen zeigten eine geeignete Bildung und Isolation von rMVA mittels Vektorplasmiden, die eine Wirstbereichsselektion auf der Basis transienter Expression des Vaccinia-Virus-K1L-Gens ermöglicht (58). Zur Verwendung dieser neuen Technik wurden die HCV-Target-Gensequenzen in MVA-Vektorplasmid pIIIdHR-P7.5 kloniert. Die resultierenden Plasmide pIIIdHR-HCV/1-742, pIIIdHR-HCV/1-661 und pIIIdHR-HCV/151-661 enthielten HCV-cDNA, die die Aminosäuren 1 bis 742, 1 bis 661 oder 151 bis 651 des HCV-Polyproteins codierten. rMVA wurde in BHK-21-Zellen gebildet, die mit MVA infiziert worden waren, und mit pIIIdHR-HCV/1-742, pIIIdHR-HCV/1-661 oder pIIIdHR-HCV/151-661 transfiziert wurden. Verdünnungsreihen der infizierten Zelllysate wurden auf RK-13-Zellen plattiert, die ein selektives Wachstum rekombinanter Viren bei transienter Expression des selektierbaren Markergens K1L ermöglichten. Nach drei Klonierungs-Passagen auf RK-13-Zellen waren nur rMVA nachweisbar, wie durch PCR-Analyse viraler DNA (Daten nicht gezeigt) bestätigt wurde, und die Virusisolate wurden in BHK-21-Zellen weiter plaque-gereinigt. Das Wachstum von rMVA in diesen Zellen war von der K1L-Genexpression unabhängig und führte zum Verlust des Markergens durch homologe Rekombination zwischen repetitiven DNA-Sequenzen, die das K1L-Gen innerhalb der viralen Genome flankierte (Figur 1A). Nach mehreren Durchläufen auf BHK-21-Zellen ergab die PCR-Analyse viraler DNA, dass die viralen Genome stabil integrierte HCV-Target-Gensequenzen, jedoch keine K1L-Gene mehr enthielten (Figur 1B). Die Amplifikation und Reinigung der erhaltenen Viren MVA-HCV/1-742, MVA-HCV/1-661 und MVA-HCV/151-661 ergaben Viruspräparate mit hohen Titern, die zur weiteren Analyse verwendet wurden.

### Synthese von HCV-Proteinen nach rMVA-Infektion

Da das erzeugte rMVA als Vektor-Impfstoff-Kandidat für die Abgabe und immunologische Charakterisierung von HCV-Antigenen untersucht werden sollte, sollte die Produktion von HCV-Proteinen bei Gewebekulturinfektion sorgfältig bestimmt werden. Sämtliche rMVA, die verschiedene codierende Sequenzen der Core-, E1- und E2-Proteine enthielten, steuerten die Synthese der HCV-Strukturproteine, wie durch SDS-PAGE-Analyse und Immunblotting von BHK-21-Zelllysaten gezeigt wurde, die 24 Std. nach der Infektion geerntet wurden (Figur 2A, B). Mönoklonale Antikörper, die gegen das HCV-Core-Protein gezüchtet wurden, ergaben spezifisch gleiche Mengen eines etwa 21 kDa Core-Proteins, hergestellt in MVA-HCV/1-742- oder MVA-HCV/1-661-infizierten Zellen, wohingegen keine solche Proteinbande nach Infektion mit MVA-HCV/151-661 oder nicht rekombinantem MVA nachgewiesen wurde (Figur 2A). An späteren Zeitpunkten und mit steigenden Mengen Core-Protein ließ sich eine zweite 23 kDa-spezifische Proteinbande nachweisen. Die Synthese von HCV-Hüllprotein wurde mit einem E2-spezifischen polyklonalen Kaninchen-Antiserum überwacht. Sämtliche drei rekombinanten Viren erzeugten Fig. 2B zufolge E2-Polypeptide, die als Proteine mit Molekulargewichten von etwa 60 kDa für MVA-HCV/1-742 und etwa 50 kDa für MVA-HCV/1-661 und MVA-HCV/151-661 sichtbar gemacht wurden. Die letzteren Viren enthalten beide Expressions-Cassetten für HCV-Polyproteine mit C-terminal verkürzten E2-Sequenzen. Mehrere andere kleinere Proteinbanden wurden ebenfalls durch das polyklonale Serum gefärbt und stellen wahrscheinlich Proteine dar, die nicht HCV-codiert sind, da sie ebenfalls in Kontrolllysaten aus Zellen nachgewiesen wurden, die mit Wildtyp-MVA infiziert waren.

Zudem sollten die Kinetiken der HCV-Proteinsyynthese bei der rMVA-Infektion überwacht werden. Wir infizierten synchron BHK-21-Zell-Monolayer mit 10 infektiösen Einheiten/Zelle MVA-HCV/1-742 und präparierten Zelllysate für die Western-Blot-Analyse zu mehreren Zeitpunkten während eines Zeitraums von zwei Tagen nach Infektion (Figur 2C, D). Bereits 4 Std. nach der Infektion konnte die Synthese von HCV-Core-Protein nachgewiesen werden (Fig. 2C), wohingegen das erste rekombinante E2-Protein (Fig. 2D) klar 8 Std. nach Infektion auftrat. Die Mengen beider rekombinanter Proteine stiegen deutlich während einer Zeitspanne von 24 Std. nach der Infektion an. Lysate von Zellen, die 24 Std. mit Wildtyp-MVA infiziert worden waren, dienten als negative Kontrollen.

### Analyse von zellassoziierten und sezernierten Formen von E2-Antigen, hergestellt von rMVA

Posttranslationale Modifikation der abgegebenen Antigene kann die Immunogenität und die Schutzkapazität von Vektor-Impfstoffen entscheidend beeinflussen. Die HCV-Hüllproteine E1 und E2 sind stark durch Glykosylierung modifiziert und sind wahrscheinlich Typ-I-Transmembran-Glykoproteine mit einer carboxyterminalen hydrophoben Anker-Domäne. Die Entfernung des E2-Transmembranankers führt zur Sekretion der E2-Ectodomäne (Übersicht siehe 17). Bei Infektion von Säugetierzellen sollte das eukaryotische MVA-Expressionssystem das Auftreten authentischer posttranslationaler Modifikationen rekombinanter Proteine bewirken. Zur Überwachung diese Prozesses wurden die E2-Proteine biochemisch charakterisiert, die bei Infektion mit rMVAs synthetisiert werden. BHK-21-Zellen wurden mit MVA-HCV/1-742, MVA-HCV/1-661 und MVA-HCV/151-661 infiziert. Gesamt-Zelllysate (Fig. 3A) oder Präzipitate von Überständen aus infizierten Zellen (Fig. 3B) wurden mit PNGase F behandelt und durch Western-Blot mittels polyklonalem anti-E2-Antiserum analysiert. Exprimierte E2-Proteine mit ~60 kDa (MVA-HCV/1-742) oder ~50 kDa (MVA-HCV/1-661 und MVA-HCV/151-661) in Zelllysaten wurden auf eine Größe von etwa 33 kDa (Volllängen-E2) bzw. 31 kDa (verkürztes E2) reduziert, entsprechend dem unglykosylierten Peptid-Gerüst von E2 (Fig. 3A). Sezerniertes E2 konnte nur nach Infektion mit den rekombinanten Viren MVA-HCV/1-661 und MVA-HCV/151-661 nachgewiesen werden, die ein in der Transmembran-Domäne verkürztes E2 produzieren. Sezerniertes E2 ist stärker modifiziert als zellassoziiertes E2 mit einem apparenten Molekulargewicht von -75 kDa. Bei Behandlung mit PNGase F werden diese Proteine auch auf eine Größe von etwa 31 kDa reduziert, entsprechend dem Peptid-Gerüst des verkürzten E2. Im nächsten Schritt wurde zudem der Glykosylierungstyp in diesen sezernierten E2-Proteinen charakterisiert. Zellen wurden mit MVA-HCV/1-661 und MVA-HCV/151-661 infiziert, Zelllysate oder Überstände infizierter Zellen wurden mit Endoglycosidase H (Fig. 4) behandelt. Intrazelluläres E2 ist zwar empfindlich gegenüber EndoH, wie durch eine Reduktion des MW von -50 kD auf -32 kD (Fig. 4A) nachgewiesen wurde, jedoch ist sezerniertes E2 weitestgehend nicht betroffen (Fig. 4B). Die E2-Banden von der PNGase F-Spaltung hatten ein niedrigeres MW als die entsprechenden Banden der Endo-H-Spaltung, da das letztere Enzym nach der Spaltung einen GlcNAc-Rest an der Seitenkette von Asn hinterlässt. Diese Beobachtung stimmt mit einer komplexeren Glycosylierung von C-terminal verkürztem E2 beim Durchtritt durch den Golgi-Apparat überein.

### Impfung von Mäusen mit rMVA ruft HCV- und Vaccinia-Virusspezifische Immunantworten hervor.

Nun wurde die Immunogenität verschiedener rMVA-Impfstoffe in Mäusen untersucht, da bekannt war, dass die Produktion des HCV-Core-Proteins durch Vaccinia-Virus-Rekombinanten eine Immununterdrückung in Mäusen vermittelt (36). Zudem sollte die mögliche Modulation der Immunantworten durch Vollängen-HCV-Core-Protein, hervorgerufen durch zwei unserer rMVA-Impfstoffe, bestimmt werden. Daher wurden HCV-spezifische humorale oder zelluläre Antworten analysiert, die gegen HCV-E1- und -E2-Antigene gerichtet waren. Nach dreimaliger intraperitonealer Immunisierung von BALB/c-Mäusen mit rekombinantem oder Wildtyp-MVA wurden HCV-E1-spezifische CTL-Antworten mit einem E1(312-326)-BALB/c-Epitop untersucht. Splenocyten, die aus Tieren stammten, die mit MVA/HCV/151-661 oder MVA-HCV/1-661 immunisiert worden waren, zeigten E1-spezifische Lyse von 75% bei einem E:T-Verhältnis von 9:1, Splenozyten von Tieren, die mit MVA-HCV/1-742 immunisiert worden waren, ergaben eine spezifische Lyse von 47%, wenn sie mit dem E1-Peptid stimuliert wurden (Fig. 5). Es wurde eine höhere E1-spezifische Lyse in Kulturen mit Splenozyten von Mäusen beobachtet, die mit rMVA-exprimierenden C-terminalen verkürzten E2-Proteinen immunisiert worden waren.

Serumproben der gleichen BALB/c-Mäuse wurden zudem auf Antikörper-Antworten gegen das HCV-E2-Protein durch ELISA untersucht (Fig. 6). Es wurden E2-spezifische Antikörper mit mittleren Titern von 1:2512 (MVA-HCV/151-661), 1:933 (MVA-HCV/1-661) und 1:176 (MVA-HCV/1-742) nachgewiesen. Es wurden höhere Mengen anti-E2-Antikörper in Tieren gefunden, die mit MVA-HCV/151-661 immunisiert worden waren, obgleich die Unterschiede unter den Gruppen nicht statistisch signifikant waren.

Das HCV-Core-Protein kann wie bereits erwähnt eine immunmodulatorische Funktion aufweisen. Nun wurde untersucht, ob die stärkere anti-E2-Antikörperantwort MVA-HCV/151-661-injizierter Mäuse der großen Verkürzung des Core-Proteins in diesem Virus zugeschrieben werden könnte. Es wurde erwartet, dass der HCV-Kern ebenfalls die Induktion der Vaccinia-Virus-MVA-spezifischen Immunantworten moduliert. Daher wurden die Mengen MVA-neutralisierender Antikörper in den gleichen Mausseren, wie sie für den. ELISA verwendet wurden, gemessen. Tabelle 1 zufolge wurde kein Unterschied der anti-MVA-Antikörperantwort unter den Gruppen, die mit rekombinantem oder Wildtyp-MVA-exprimierendem Volllängen- oder verkürztem Core-Protein immunisiert worden waren, entdeckt.

### Zusammenfassung der Experimente

Vorhergehende Experimente etablierten Daten zur Immunogenität und **Schutzwirkung** rekombinanter MVA-Impfstoffe, die virale Antigene abgeben, bei einer Reihe von Tier-Modellsystemen (61, 29, 27, 64, 65, 44, 59). Es bestand aber immer noch ein dringender Bedarf an einem Impfstoff gegen HCV-Infektion, und es mußte ein Impfstoff-Kandidat mit ausreichender **immunogener und möglicherweise schützender Kapazität** identifiziert werden. Zur Untersuchung, der Eignung von MVA zur Produktion von HCV-Antigenen wurde eine Reihe von **rMVA mit eingesetzten Expressionskassetten von HCV-Gensequenzen** konstruiert, die für die Strukturproteine C-E1-**E2** codieren. Wir wollten die Eigenschaften von rekombinantem MVA bestimmen, das so ausgelegt war, dass HCV-E1-E2-Hüllproteine oder sezerniertes E2-Antigen mit oder ohne Volllängen-HCV- Kapsid/Core-Protein produziert wurden. Die Bildung und Isolierung von rMVA beruhte auf der transienten Wirtsbereichs-Genselektion (58) und zeigte, dass sich diese Technik besonders gut eignet, wenn mehrere MVA-Vektor-Viren zur vergleichenden Untersuchung erzeugt werden müssen. Rekombinantes MVA, das die verschiedenen HCV-codierenden Sequenzen unter der Kontrolle des Vaccinia-Virus-Early-Late-Promotors P7.5 exprimiert, konnte leicht erhalten werden, und anfängliche Experimente zeigten, dass sämtliche Konstrukte übereinstimmend ähnliche Mengen an HCV-Strukturproteinen produzierten.

Die HCV-Core-Sequenz ist eine der am stärksten konservierten Bereiche im viralen Genom, die bis zu Viren aus verschiedenen HCV-Genotypen reicht und das Core-Protein als Antigen zur Immunisierung interessant macht (6, 57, 7). Trotzdem ist der Einschluss von Core in Impfstoff-Kandidaten stark umstritten, da seine multifunktionellen Eigenschaften möglicherweise an der Regulation der Wirtszell-Apoptose, Transkription, Transformation und Immunpräsentation beteiligt sind (43, 4). Die Expression von HCV-Sequenzen, die Polyprotein AS 1-742 und 1-661 codieren, durch rekombinantes MVA führte überwiegend zur Synthese der 21-kDa-Core-Proteine (Fig. 2A). Nur zu späteren Zeitpunkten der Infektion, bei steigenden Mengen an produziertem rekombinantem Protein, wurde eine zweite core-spezifische Proteinbande bei 23 kDa Größe sichtbar. Das 23 kDa-Protein ist wahrscheinlich das unreife Core-Protein mit 191 Aminosäuren, das aus einer anfänglichen Polyproteinspaltung hervorgeht, wohingegen das 21 kDa-Protein wahrscheinlich das reife Core-Protein darstellt, das in Viruspartikeln zugegen ist (28, 26, 51, 55, 66). Diese Daten zeigen eine effiziente frühe Synthese und eine Prozessierung der Core-Polypeptide in MVA-HCV-Vektorinfizierten Zellen.

Nach der Infektion mit MVA-HCV/1-742 wurde die Synthese eines etwa 60 kDa E2-Proteins bestätigt, das strikt zellassoziiert blieb (Fig. 2B). Die Anreicherung eines 75 kDa C-terminal verkürzten E2-Proteins in Zellkulturüberständen ließ auf die Funktionalität des sekretorischen Wegs in MVA-HCV-infizierten Zellen schließen. Der Vergleich von zellassoziiertem und sezerniertem verkürzten E2, produziert von MVA-HCV/1-661 und MVA-HCV/151-661, durch SDS-PAGE (Figur 3AB) zeigte, dass die sezernierte Form langsamer wanderte. Dies erfolgte aufgrund unterschiedlicher Glykosylierung. Entsprechend wandern beide E2-Proteine nach Deglykosylierung mit PGNAse F auf gleicher Höhe. Zudem wurde sezerniertes E2 unempfindlich gegenüber einer Behandlung mit Endoglycosidase H, was eindeutig die Aufnahme komplexer Zucker beim Transport durch den Golgi-Apparat zeigt. Die Expression des HCV-E1-Proteins wurde zwar nicht formal analysiert, jedoch bildet das E2-Protein einen stromabwärts gelegenen Teil des HCV-Polyproteins und die Demonstration der E2-Synthese lässt daher auf die Produktion der E1-Proteinsequenzen schließen. Die Richtigkeit dieses Gedankens wurde durch den Nachweis E1-spezifischer T-Zellantworten nach der Impfung mit rekombinanten MVA-HCV-Viren bestätigt.

Zur vergleichenden Untersuchung der Immunogenität der Impfstoffe wurden BALB/c-Mäuse geimpft und auf die Induktion von Immunantworten untersucht, die direkt gegen HCV-Hüllproteine gerichtet waren, die von allen Vektor-Viren erzeugt wurden. Für die Hüll-Antigene E1 und E2 spezifische CTL sind in der Leber und im peripheren Blut HCV-infizierter Menschen und Schimpansen zugegen (Walker 1996, Sem. Virol.). Neuere Daten der Untersuchung von Immunantworten in HCVinfizierten Schimpansen lassen darauf schließen, dass CD8+CTL mit einer akuten Auflösung der Infektion einher gehen (12). Die Analyse der Fein-Spezifität lang beständiger CTL ergab neun verschiedene Peptidepitope der HCV-Proteine E1, E2, P7, NS2, NS3, NS5a, wobei vier dieser Epitope in E1/E2-Sequenzen vorkommen. Das E1-Epitop 312-326 wurde erfolgreich zur In-vitro-Restimulation von Splenozyten aus geimpften BALB/c-Mäusen verwendet, und es wurden epitopspezifische CTL nachgewiesen, die von allen drei rMVA-Impfstoffen induziert wurden. Interessanterweise war der Nachweis der E1-spezifischen CTL auch mehr als 12 Wochen nach der letzten Immunisierung möglich. Dies legt nahe, dass die Immunisierungen über einen langen Zeitraum beständige (Memory-Phase) E1-spezifische T-Zellantworten induzieren konnten.
Die Cosynthese des HCV-Core-Proteins beeinflusste die Induktion E1-spezifischer CTL-Antworten nicht, jedoch schien eine Immunisierung mit Vektor-Viren, die C-terminal verkürzte sezernierte E2-Proteine produzierten, höhere E1-spezifische cytotoxische Aktivitäten hervor zu rufen. Die Glykoproteine E1 und E2 bilden Komplexe, die im Endoplasmatischen Reticulum (ER) aufgrund der Wechselwirkung hydrophober Transmembransequenzen an den C-Termini der Proteine zurückgehalten werden (10). Die Entfernung oder Mutation der E2-Transmembran-Region hemmt die Bildung nativer E1-E2-Komplexe und verhindert die korrekte Faltung des E1-Glycoproteins (11, 48). Für eine Präsentation durch MHC-I-Moleküle scheint das E1-Antigen aus dem Endoplasmatischen Reticulum zu stammen, erfordert eine cytoplasmatische Prozessierung (54) und beruht wahrscheinlich auf einem sekundären Exportweg für defekte Transmembran-Glycoproteine ins Cytosol, wie es für das Influenza-Nucleoprotein oder die zelluläre Glycoprotein-Tyrosinase vorgeschlagen wurde (2, 47). Aufgrund dieses Befunds der höheren CTL-Aktivität nach der Immunisierung mit Vektor-Viren, die sezerniertes E2 produzieren, ist man versucht zu spekulieren, dass die Mobilisierung von verkürztem E2 für den sekretorischen Weg E1 vorzugsweise für den obigen Prozessierungs-Weg verfügbar macht und eine verstärkte MHC-I-restringierte Präsentation von E1-Antigen ermöglicht.

Der Schutz, der nach der prophylaktischen Impfung mit rekombinanten HCV-Hüll-Antigenen im Schimpansenmodell erzielt wurde, wurde mit E2-spezifischen Antikörpern korreliert (9). Folglich wurde dem E2-Protein große Aufmerksamkeit als Impfstoff-Antigen-Kandidat zur Induktion HCVneutralisierender Antikörper geschenkt (68, 56). Bei der Untersuchung auf E2-spezifische humorale Immunantworten in den Experimenten wurden spezifische Antikörper entdeckt, die nach der Immunisierung mit allen drei rMVA-Imfstoffen erzeugt wurden. Höhere Antikörper-Titer wurden in Seren aus Tieren erhalten, die mit Vektoren geimpft wurden, die sezernierte E2-Proteine mit verkürzter Transmembran-Domäne synthetisieren. Die Sekretion von E2 könnte natürlich bessere Antikörper-Antworten hervorrufen, wohingegen die Wechselwirkung der Vollängen-E1- und E2-Proteine zur Bildung intrazellulär zurückgehaltener Komplexe wichtige Epitope maskieren und eine optimale Immunpräsentation verhindern könnte. Das HCV-Core-Protein, hergestellt durch replikationskompetentes Vaccinia-Virus, scheint Wirts-Immunantworten zu supprimieren, insbesondere die Erzeugung von Vaccinia-Virus-spezifischen Immunantworten unterdrücken zu können (Large et al 1999, J Immunol 162:931-938). Zu diesen Daten passend schienen in den hier gezeigten Impfversuchen die besten E2-spezifischen Antikörper-Antworten durch den MVA-HCV/151-661-Impfstoff induziert zu werden, welcher verkürztes E2 in Abwesenheit von Core-Antigen produzierte. Die eingehende Analyse der Vaccinia-Virus-spezifischen neutralisierenden Antikörper-Antworten zeigte aber, dass von allen rMVA-HCV-Impfstoffen nahezu gleichartige Mengen an Vaccinia-spezifischer Immunität hervorgerufen wurde. Die Bewertung der Ergebnisse aller Immunisierungsversuche lässt daher schlussfolgern, dass das HCV-Core-Antigen (zumindest in den vorliegenden Experimenten) mit replikationsdefizientem Vaccinia-Virus MVA als Expressionsvektor keine nachweisbare immunmodulierende Wirkung hatte.

Diese Untersuchung beweist zusammengefasst, dass rMVA zur effizienten Produktion posttranslational gereifter Core- und Hüllproteine verwendet werden kann. Die Impfung von BALB/c-Mäusen mit rMVA-Impfstoffen induzierte darüber hinaus vergleichbare Mengen E2-spezifischer Antikörper und lang dauernde E1-spezifische cyctotoxische T-Zell-Antworten und belegt die Verwendbarkeit dieses viralen Vektors für die weitere Bewertung als viel versprechenden Impfstoff gegen HCV-Infektion.

Erfindungsgemäß wurde somit rMVA als potentieller Impfstoffkandidat gegen eine HCV-Infektion untersucht. Im ersten Schritt wurden MVA-Vektor-Impfstoffe konstruiert, welche sämtliche Haupt-HCV-Virionkomponenten codierenden Sequenzen exprimieren, um die Synthese rekombinanter HCV-Proteine bei MVA-Infektion zu charakterisieren, und um ihre Immunogenität beim Gebrauch als Impfstoffe zu bewerten.

Bei dieser Untersuchung wurde eine starke Synthese und eine effiziente posttranslationale Reifung von Vollängen- oder verkürzten HCV-Strukturproteinen nachgewiesen, die bei In-vitro-Infektion mit rMVA hergestellt wurden. Eine Induktion von HCV-E1- und E2-antigenspezifischen humoralen oder zellulären Immunantworten in BALB/c-Mäusen zur rMVA-Immunisierung konnte gezeigt werden. Sämtliche Impfstoffe riefen ausgeglichene Mengen Vaccinia-Virus-spezifischer zirkulierender Antikörper hervor. Die vorliegenden Daten lassen auf eine hohe Immunogenität rMVA-exprimierter HCV-Strukturantigene, einschließlich des vollständigen HCV-Core-Antigens, schließen.

**Tabelle 1: Vaccinia-Virus-neutralisierende Antikörper, induziert durch MVA-Impfstoffe**

| **Impfstoff^{a}** | **Titer^{b} (reziproker mittlerer log2)^{c}** | | | |
|---|---|---|---|---|
| | **d0** | **d28** | **d55** | **d110** |
| MVA | <5,3 | 10,3 | 12,3 | 12,3 |
| MVA-HCV/151-661 | <5,3 | 9, 5 | 12,3 | 12,3 |
| MVA-HCV/1-661 | <5,3 | 10, 1 | 11,9 | 12,1 |
| MVA-HCV/1-742 | <5,3 | 9,9 | 12,3 | 12,3 |

| | | | | |
|---|---|---|---|---|
| a) 10⁸ IU MVA-Impfstoff, gegeben auf dem intraperitonealen Weg an den Tagen 0, 38, 81 b) Serumantikörpertiter wurden bestimmt durch den 50% Vaccinia-Virus-Plaque-Reduktionstest c) n = 5, Maximale Standardabweichung ist | | | | |

### Literatur:

1. Amara, R. R., F. Villinger, J. D. Altman, S. L. Lydy, O. N. SP, S. I. Staprans, D. C. Montefiori, Y. Xu, J. G. Herndon, L. S. Wyatt, M. A. Candido, N. L. Kozyr, P. L. Earl, J. M. Smith, H. L. Ma, B. D. Grimm, M. L. Hulsey, J. Miller, H. M. McClure, J. M. McNicholl, B. Moss, and H. L. Robinson 2001. Control of a Mucosal Challenge and Prevention of AIDS by a Multiprotein DNA/MVA Vaccine Science. 292:69-74.
2. Bacik, I., H. L. Snyder, L. C. Anton, G. Russ, W. Chen, J. R. Bennink, L. Urge, L. Otvos, B. Dudkowska, L. Eisenlohr, and J. W. Yewdell 1997. Introduction of a glycosylation site into a secreted protein provides evidence for an alternative antigen processing pathway: transport of precursors of major histocompatibility complex class I-restricted peptides from the endoplasmic reticulum to the cytosol J Exp Med. 186:479-87.
3. Belyakov, I. M., L. S. Wyatt, J. D. Ahlers, P. Earl, C. D. Pendleton, B. L. Kelsall, W. Strober, B. Moss, and J. A. Berzofsky 1998. Induction of a mucosal cytotoxic T-lymphocyte response by intrarectal immunization with a replication-deficient recombinant vaccinia virus expressing human immunodeficiency virus 89.6 envelope protein J Virol. 72:8264-72.
4. Bergqvist, A., and C. M. Rice 2001. Transcriptional activation of the interleukin-2 promoter by hepatitis C virus core protein J Virol. 75:772-81.
5. Bruna-Romero, O., J. J. Lasarte, G. Wilkinson, K. Grace, B. Clarke, F. Borras-Cuesta, and J. Prieto 1997 induction of cytotoxic T-cell response against hepatitis C virus structural antigens using a defective recombinant adenovirus Hepatology. 25:470-7.
6. Bukh, J., R. H. Purcell, and R. H. Miller 1994. Sequence analysis of the core gene of 14 hepatitis C virus genotypes Proc Natl Acad Sei U S A. 91:8239-43.
7. Cerny, A., J. G. McHutchison, C. Pasquinelli, M. E. Brown, M. A. Brothers, B. Grabscheid, P. Fowler, M. Houghton, and F. V. Chisari 1995. Cytotoxic T lymphocyte response to hepatitis C virus-derived peptides containing the HLA A2.1 binding motif J Clin Invest. 95:521-30.
8. Choo, Q. L., G. Kuo, A. J. Weiner, L. R. Overby, D. W. Bradley, and M. Houghton 1989. Isolation of a cDNA clone derived from a bloodborne non-A, non-B viral hepatitis genome Science. 244:359-62.
9. Choo, Q. L., G. Kuo, R. Ralston, A. Weiner, D. Chien, G. Van Nest, J. Han, K. Berger, K. Thudium, C. Kuo, and et al. 1994. Vaccination of chimpanzees against infection by the hepatitis C virus Proc Natl Acad Sci U S A. 91:1294-8.
10. Cocquerel, L., J. C. Meunier, A. Pillez, C. Wychowski, and J. Dubuisson 1998. A retention signal necessary and sufficient for endoplasmic reticulum localization maps to the transmembrane domain of hepatitis C virus glycoprotein E2 J Virol. 72:2183-91.
11. Cocquerel, L., C. Wychowski, F. Minner, F. Penin, and J. Dubuisson 2000. Charged residues in the transmembrane domains of hepatitis C virus glycoproteins play a major role in the processing, subcellular localization, and assembly of these envelope proteins J Virol. 74:3623-33.
12. Cooper, S., A. L. Erickson, E. J. Adams, J. Kansopon, A. J. Weiner, D. Y. Chien, M. Houghton, P. Parham, and C. M. Walker 1999. Analysis of a successful immune response against hepatitis C virus Immunity. 10:439-49.
13. Davis, G. L., D. R. Nelson, and G. R. Reyes 1999. Future options for the management of hepatitis C Semin Liver Dis. 19:103-12.
14. Dietzschold, B., H. H. Wang, C. E. Rupprecht, E. Celis, M. Tollis, H. Ertl, E. Heber-Katz, and H. Koprowski 1987. Induction of protective immunity against rabies by immunization with rabies virus ribonucleoprotein Proc Natl Acad Sci U S A. 84:9165-9.
15. Drexler, I., E. Antunes, M. Schmitz, T. Wolfel, C. Huber, V. Erfle, P. Rieber, M. Theobald, and G. Sutter 1999. Modified vaccinia virus Ankara for delivery of human tyrosinase as melanoma-associated antigen: induction of tyrosinase- and melanoma- specific human leukocyte antigen A*0201-restricted cytotoxic T cells in vitro and in vivo Cancer Res. 59:4955-63.
16. Drexler, I., K. Heller, B. Wahren, V. Erfle, and G. Sutter 1998. Highly attenuated modified vaccinia virus Ankara replicates in baby hamster kidney cells, a potential host for virus propagation, but not in various human transformed and primary cells J Gen Virol. 79:347-52.
17. Dubuisson, J. 2000. Folding, assembly and subcellular localization of hepatitis C virus glycoproteins Curr Top Microbiol Immunol. 242:135-48.
18. Dubuisson, J., H. H. Hsu, R. C. Cheung, H. B. Greenberg, D. G. Russell, and C. M. Rice 1994. Formation and intracellular localization of hepatitis C virus envelope glycoprotein complexes expressed by recombinant vaccinia and Sindbis viruses J Virol. 68:6147-60.
19. Duvet, S., L. Cocquerel, A. Pillez, R. Cacan, A. Verbert, D. Moradpour, C. Wychowski, and J. Dubuisson 1998. Hepatitis C virus glycoprotein complex localization in the endoplasmic reticulum involves a determinant for retention and not retrieval J Biol Chem. 273:32088-95.
20. Farci, P., A. Shimoda, D. Wong, T. Cabezon, D. De Gioannis, A. Strazzera, Y. Shimizu, M. Shapiro, H. J. Alter, and R. H. Purcell 1996. Prevention of hepatitis C virus infection in chimpanzees by hyperimmune serum against the hypervariable region 1 of the envelope 2 protein Proc Natl Acad Sci U S A. 93:15394-9.
21. Flint, M., J. Dubuisson, C. Maidens, R. Harrop, G. R. Guile, P. Borrow, and J. A. McKeating 2000. Functional characterization of intracellular and secreted forms of a truncated hepatitis C virus E2 glycoprotein J Virol. 74:702-9.
22. Fournillier, A., E. Depla, P. Karayiannis, O. Vidalin, G. Maertens, C. Trepo, and G. Inchauspe 1999. Expression of noncovalent hepatitis C virus envelope E1-E2 complexes is not required for the induction of antibodies with neutralizing properties following DNA immunization J Virol. 73:7497-504.
23. Geissler, M., K. Tokushige, T. Wakita, V. R. Zurawski, and J. R. Wands 1998. Differential cellular and humoral immune responses to HCV core and HBV envelope proteins after genetic immunizations using chimeric constructs Vaccine. 16:857-67.
24. Gish, R. G. 1999. Standards of treatment in chronic hepatitis C Semin Liver Dis. 19:35-47.
25. Gordon, E. J., R. Bhat, Q. Liu, Y. F. Wang, C. Tackney, and A. M. Prince 2000. Immune responses to hepatitis C virus structural and nonstructural proteins induced by plasmid DNA immunizations J Infect Dis. 181:42-50.
26. Grakoui, A., C. Wychowski, C. Lin, S. M. Feinstone, and C. M. Rice 1993. Expression and identification of hepatitis C virus polyprotein cleavage products J Virol. 67:1385-95.
27. Hanke, T., T. J. Blanchard, J. Schneider, G. S. Ogg, R. Tan, M. Becker, S. C. Gilbert, A. V. Hill, G. L. Smith, and A. McMichael 1998. lmmunogenicities of intravenous and intramuscular administrations of modified vaccinia virus Ankara-based multi-CTL epitope vaccine for human immunodeficiency virus type 1 in mice J Gen Virol. 79:83-90.
28. Harada, S., Y. Watanabe, K. Takeuchi, T. Suzuki, T. Katayama, Y. Takebe, I. Saito, and T. Miyamura 1991. Expression of processed core protein of hepatitis C virus in mammalian cells J Virol. 65:3015-21.
29. Hirsch, V. M., T. R. Fuerst, G. Sutter, M. W. Carroll, L. C. Yang, S. Goldstein, M. Piatak, Jr., W. R. Elkins, W. G. Alvord, D. C. Montefiori, B. Moss, and J. D. Lifson 1996. Patterns of viral replication correlate with outcome in simian immunodeficiency virus (SIV)-infected macaques: effect of prior immunization with a trivalent SIV vaccine in modified vaccinia virus Ankara J Virol. 70:3741-52.
30. Hollinger 1990. Non-A, non-B hepatitis viruses. Raven Press Ltd, New York.
31. Hoofnagle, J. H., and A. M. di Bisceglie 1997. The treatment of chronic viral hepatitis N Engl J Med. 336:347-56.
32. Inchauspe, G., L. Vitvitski, M. E. Major, G. Jung, U. Spengler, M. Maisonnas, and C. Trepo 1997. Plasmid DNA expressing a secreted or a nonsecreted form of hepatitis C virus nucleocapsid: comparative studies of antibody and T-helper responses following genetic immunization DNA Cell Biol. 16:185-95.
33. Iwarson, S., E. Tabor, H. C. Thomas, P. Snoy, and R. J. Gerety 1985. Protection against hepatitis B virus infection by immunization with hepatitis B core antigen Gastroenterology. 88:763-7.
34. Kato, N., Y. Ootsuyama, H. Sekiya, S. Ohkoshi, T. Nakazawa, M. Hijikata, and K. Shimotohno 1994. Genetic drift in hypervariable region 1 of the viral genome in persistent hepatitis C virus infection J Virol. 68:4776-84.
35. Kuo, G., Q. L. Choo, H. J. Alter, G. L. Gitnick, A. G. Redeker, R. H. Purcell, T. Miyamura, J. L. Dienstag, M. J. Alter, C. E. Stevens, and et al. 1989. An assay for circulating antibodies to a major etiologic virus of human non-A, non-B hepatitis Science. 244:362-4.
36. Large, M. K., D. J. Kittlesen, and Y. S. Hahn 1999. Suppression of host immune response by the core protein of hepatitis C virus: possible implications for hepatitis C virus persistence J Immunol. 162:931-8.
37. Lasarte, J. J., F. J. Corrales, N. Casares, A. Lopez-Diaz de Cerio, C. Qian, X. Xie, F. Borras-Cuesta, and J. Pristo 1999. Different doses of adenoviral vector expressing IL-12 enhance or depress the immune response to a coadministered antigen: the role of nitric oxide J Immunol. 162:5270-7.
38. Li, L., J. He, and L. Zhao 1998. [Epidemiologic features of viral hepatitis in Fujian] Zhonghua Liu Xing Bing Xue Za Zhi. 19:89-92.
39. Liang, T. J. 1998. Combination therapy for hepatitis C infection N Engl J Med. 339:1549-50.
40. Llang, T. J., B. Rehermann, L. B. Seeff, and J. H. Hoofnagie 2000. Pathogenesis, natural history, treatment, and prevention of hepatitis C Ann Intern Med. 132:296-305.
41. Makimura, M., S. Miyake, N. Akino, K. Takamori, Y. Matsuura, T. Miyamura, and I. Saito 1996. Induction of antibodies against structural proteins of hepatitis C virus in mice using recombinant adenovirus Vaccine. 14:28-36.
42. Mayr, A., H. Stickl, H. K. Muller, K. Danner, and H. Singer 1978. [The smallpox vaccination strain MVA: marker, genetic structure, experience gained with the parenteral vaccination and behavior in organisms with a debilitated defence mechanism (author's transl)] Zentralbl Bakteriol [B]. 167:375-90.
43. McLauchlan, J. 2000. Properties of the hepatitis C virus core protein: a structural protein that modulates cellular processes J Viral Hepat. 7:2-14.
44. Men, R., L. Wyatt, I. Tokimatsu, S. Arakaki, G. Shameem, R. Elkins, R. Chanock, B. Moss, and C. J. Lai 2000. Immunization of rhesus monkeys with a recombinant of modified vaccinia virus Ankara expressing a truncated envelope glycoprotein of dengue type 2 virus induced resistance to dengue type 2 virus challenge Vaccine. 18:3113-22.
45. Meyer, H., G. Sutter, and A. Mayr 1991. Mapping of deletions in the genome of the highly attenuated vaccinia virus MVA and their influence on virulence J Gen Virol. 72:1031-8.
46. Michalak, J. P., C. Wychowski, A. Choukhi, J. C. Meunier, S. Ung, C. M. Rice, and J. Dubuisson 1997. Characterization of truncated forms of hepatitis C virus glycoproteins J Gen Virol. 78:2299-306.
47. Mosse, C. A., L. Meadows, C. J. Luckey, D. J. Kittlesen, E. L. Huczko, C. L. Slingluff, J. Shabanowitz, D. F. Hunt, and V. H. Engelhard 1998. The class I antigen-processing pathway for the membrane protein tyrosinase involves translation in the endoplasmic reticulum and processing in the cytosol J Exp Med. 187:37-48.
48. Patel, J., A. H. Patel, and J. McLauchlan 2001. The transmembrane domain of the hepatitis C virus E2 glycoprotein is required for correct folding of the E1 glycoprotein and native complex formation Virology. 279:58-68.
49. Ralston, R., K. Thudium, K. Berger, C. Kuo, B. Gervase, J. Hall, M. Selby, G. Kuo, M. Houghton, and Q. L. Choo 1993. Characterization of hepatitis C virus envelope glycoprotein complexes expressed by recombinant vaccinia viruses J Virol. 67:6753-61.
50. Ramirez, J. C., M. M. Gherardi, and M. Esteban 2000. Biology of attenuated modified vaccinia virus Ankara recombinant vector in mice: virus fate and activation of B- and T-cell immune responses in comparison with the Western Reserve strain and advantages as a vaccine J Virol. 74:923-33.
51. Santolini, E., G. Migliaccio, and N. La Monica 1994. Biosynthesis and biochemical properties of the hepatitis C virus core protein J Virol. 68:3631-41.
52. Schneider, J., S. C. Gilbert, T. J. Blanchard, T. Hanke, K. J. Robson, C. M. Hannan, M. Becker, R. Sinden, G. L. Smith, and A. V. Hill 1998. Enhanced immunogeriidty for CD8+ T cell induction and complete protective efficacy of malaria DNA vaccination by boosting with modified vaccinia virus Ankara Nat Med. 4:397-402.
53. Seef, L. B. 1999. Natural history of hepatitis C The American Journal of Medicine. 107:10-15.
54. Selby, M., A. Erickson, C. Dong, S. Cooper, P. Parham, M. Houghton, and C. M. Walker 1999. Hepatitis C virus envelope glycoprotein E1 originates in the endoplasmic reticulum and requires cytoplasmic processing for presentation by class I MHC molecules J Immunol. 162:669-76.
55. Selby, M. J., Q. L. Choo, K. Berger, G. Kuo, E. Glazer, M. Eckart, C. Lee, D. Chien, C. Kuo, and M. Houghton 1993. Expression, identification and subcellular localization of the proteins encoded by the hepatitis C viral genome J Gen Virol. 74:1103-13.
56. Shimizu, Y. K., H. Igarashi, T. Kiyohara, T. Cabezon, P. Farci, R. H. Purcell, and H. Yoshikura 1996. A hyperimmune serum against a synthetic peptide corresponding to the hypervariable region 1 of hepatitis C virus can prevent viral infection in cell cultures Virnlngy. 223:409-12.
57. Shirai, M., H. Okada, M. Nishioka, T. Akatsuka, C. Wychowski, R. Houghten, C. D. Pendleton, S. M. Feinstone, and J. A. Berzofsky 1994. An epitope in hepatitis C virus core region recognized by cytotoxic T cells in mice and humans J Virol. 68:3334-42.
58. Staib, C., I. Drexler, M. Ohlmann, S. Wintersperger, V. Erfle, and G. Sutter 2000. Transient host range selection for genetic engineering of modified vaccinia virus Ankara Biotechniques. 28:1137-42, 1144-6, 1148.
59. Stittelaar, K. J., L. S. Wyatt, R. L. de Swart, H. W. Vos, J. Groen, G. van Amerongen, R. S. van Binnendijk, S. Rozenblatt, B. Moss, and A. D. Osterhaus 2000. Protective immunity in macaques vaccinated with a modified vaccinia virus Ankara-based measles virus vaccine in the presence of passively acquired antibodies J Virol. 74:4236-43.
60. Sutter, G., and B. Moss 1992. Nonreplicating vaccinia vector efficiently expresses recombinant genes Proc Natl Acad Sei U S A. 89:10847-51.
61. Sutter, G., L. S. Wyatt, P. L. Foley, J. R. Bennink, and B. Moss 1994. A recombinant vector derived from the host range-restricted and highly attenuated MVA strain of vaccinia virus stimulates protective immunity in mice to influenza virus Vaccine. 12:1032-40.
62. Ulmer, J. B., J. J. Donnelly, S. E. Parker, G. H. Rhodes, P. L. Felgner, V. J. Dwarki, S. H. Gromkowski, R. R. Deck, C. M. DeWitt, A. Friedman, and et al. 1993. Heterologous protection against influenza by injection of DNA encoding a viral protein Science 259:1745-9.
63. Wang, Y., H. Okamoto, F. Tsuda, R. Nagayama, Q. M. Tao, and S. Mishiro 1993. Prevalence, genotypes, and an isolate (HC-C2) of hepatitis C virus in Chinese patients with liver disease J Med Virol. 40:254-60.
64. Wyatt, L. S., S. T. Shors, B. R. Murphy, and B. Moss 1996. Development of a replication-deficient recombinant vaccinia virus vaccine effective against parainfluenza virus 3 infection in an animal model Vaccine. 14:1451-8.
65. Wyatt, L. S., S. S. Whitehead, K. A. Venanzi, B. R. Murphy, and B. Moss 1999. Priming and boosting immunity to respiratory syncytial virus by recombinant replication-defective vaccinia virus MVA Vaccine. 18:392-7.
66. Yasui, K., T. Wakita, K. Tsukiyama-Kohara, S. I. Funahashi, M. Ichikawa, T. Kajita, D. Moradpour, J. R. Wands, and M. Kohara 1998. The native form and maturation process of hepatitis C virus core protein J Virol. 72:6048-55.
67. Zibert, A., H. Meisel, W. Kraas, A. Schulz, G. Jung, and M. Roggendorf 1997. Early antibody response against hypervariable region 1 is associated with acute self-limiting infections of hepatitis C virus Hepatology. 25:1245-9.
68. Zibert, A., E. Schreier, and M. Roggendorf 1995. Antibodies in human sera specific to hypervariable region 1 of hepatitis C virus can block viral attachment Virology. 208:653-61.

## Patentansprüche

1. Rekombinantes, modifiziertes Vaccinia-Virus Ankara (MVA),
**dadurch gekennzeichnet, dass**
es DNA-Sequenzen enthält, die für die HCV-Strukturantigene des Kapsid-Proteins Core und/oder des Hüll-Glycoproteins E1 und/oder des Hüll-Glycoproteins E2 des Hepatitis C-Virus (HCV), für immunogene Abschnitte oder für Epitope der genannnten HCV-Strukturantigene kodieren.

2. Rekombinantes MVA nach Anspruch 1,
**dadurch gekennzeichnet, dass**
es als DNA-Sequenzen die für E1 und E2 kodierenden Sequenzen enthält.

3. Rekombinantes MVA nach Anspruch 1 oder 2,
**dadurch gekennzeichnet, dass**
die für E2 kodierenden DNA-Sequenzen für eine mutierte Form von E2 kodieren.

4. Rekombinantes MVA nach Anspruch 3,
**dadurch gekennzeichnet, dass**
die mutierte Form von E2 die sezernierbare Form ist, in der zumindest ein Teil des lipophilen Anteils von E2 deletiert ist.

5. Rekombinantes MVA nach einem oder mehreren der vorhergehenden Ansprüche,
**dadurch gekennzeichnet, dass**
die DNA-Sequenzen in nicht-essentiellen Bereichen im MVA-Genom integriert sind.

6. Rekombinantes MVA nach einem oder mehreren der vorhergehenden Ansprüche,
**dadurch gekennzeichnet, dass**
die DNA-Sequenzen in Bereichen natürlich vorkommender Deletionen im MVA-Genom integriert sind.

7. Rekombinantes MVA nach Anspruch 6,
**dadurch gekennzeichnet, dass**
die Stelle der natürlich vorkommenden Deletion die Deletion III oder eine andere Deletion in einem nicht-essentiellen Bereich des MVA-Genoms ist.

8. Rekombinantes MVA nach einem oder mehreren der vorhergehenden Ansprüche,
**dadurch gekennzeichnet, dass**
die DNA-Sequenzen unter Transkriptions-Kontrolle Vacciniavirus-spezifischer Promotoren und/oder unter Kontrolle nicht Vaccinia-Virus eigener Promotoren stehen, bevorzugt in Verbindung mit weiteren Kontrollsequenzen wie Enhancerelementen.

9. Rekombinantes MVA nach Anspruch 8,
**dadurch gekennzeichnet, dass**
die DNA-Sequenzen unter Transkriptions-Kontrolle des Vacciniavirus-spezifischen Early/Late Promotors P7.5 stehen

10. Rekombinantes MVA nach einem oder mehreren der vorhergehenden Ansprüche,
**dadurch gekennzeichnet, dass**
E1 und E2 zur Bildung von Heterodimeren nicht befähigt sind.

11. Pharmazeutische Zubereitung,
**dadurch gekennzeichnet, dass**
sie zumindest ein rekombinantes MVA nach einem oder mehreren der vorhergehenden Ansprüche 1 - 10 und pharmazeutisch verträgliche Träger- und Hilfsstoffe enthält.

12. Pharmazeutische Zubereitung nach Anspruch 11,
**dadurch gekennzeichnet, dass**
sie in Form einer Vakzine vorliegt.

13. Isolierte Eukaryontenzelle,
**dadurch gekennzeichnet, dass**
sie ein rekombinantes MVA nach einem oder mehreren der Ansprüche 1-10 enthält.

14. Eukaryontenzelle nach Anspruch 13,
**dadurch gekennzeichnet, dass**
die Zelle eine Hühnerembryofibroblastenzelle oder eine Babyhamster-Nierenzelle, bevorzugt BHK-21, oder eine Antigen-präsentierende Zelle, bevorzugt eine dendritische Zelle, ist.

15. Verwendung von rekombinantem MVA nach einem oder mehreren der Ansprüche 1 -10 zur Herstellung eines Arzneimittels zur Therapie und Prophylaxe von HCV Infektionen und hierdurch verursachter Erkrankungen, insbesondere chronischer Lebererkrankungen und Lebertumoren.

16. Verwendung von rekombinantem MVA nach Anspruch 15 zur Herstellung eines Arzneimittels, enthaltend weiterhin zumindest pharmazeutisch verträgliche Träger- und Hilfsstoffe.

17. Verwendung von rekombinantem MVA nach einem oder mehreren der Ansprüche 1 - 10 zur Herstellung einer Vakzine, zur Produktion von rekombinanten HCV-Strukturproteinen oder zur Herstellung das rekombinante HCV-Strukturprotein produzierender Eukaryontenzellen.

18. Verfahren zur Herstellung von rekombinanten HCV-Struktur-Polypeptiden oder funktioneller Teile hiervon mit den nachfolgenden Schritten:
(a) Kultivieren von Zellen nach Anspruch 13 oder 14 unter geeigneten Bedingungen, und
(b) Exprimieren, Isolieren und wahlweise Aufreinigen der rekombinanten HCV-Struktur-Polypeptide, immunogenen Abschnitten hiervon oder Epitopen hiervon.

19. Verfahren zur Herstellung von rekombinantem modifiziertem Vaccinia-Virus Ankara(MVA) mit den nachfolgenden Schritten:
(a) Einbringen von DNA Sequenzen, die für die Strukturantigene für das Kapsid-Protein Core und/oder das Hüll-Glycoprotein E1 und/oder das Hüll-Glycoprotein E2 des Hepatitis C-Virus (HCV) kodieren, oder immunogener Abschnitte oder Epitope hiervon in einen nicht-essentiellen Bereich eines MVA-Vektors zur Herstellung eines rekombinanten MVA-Vektors;
(b) Einbringen des rekombinanten MVA-Vektors in eine Eukaryontenzelle und Vermehren des Vektors in dieser Zelle; und
(c) wahlweise Isolieren der Virusteilchen oder deren DNA oder RNA.

20. DNA oder die hieraus transkribierte RNA des rekombinanten MVA nach einem oder mehreren der vorhergehenden Ansprüche 1 - 10, bevorzugt in isolierter Form, wobei die DNA oder die RNA das rekombinante MVA umfasst, einschließlich der DNA, die für die HCV-Strukturantigene des Kapsid-Proteins Core und/oder des Hüll-Glycoproteins E1 und/oder des Hüll-Glycoproteins E2 des Hepatitis C-Virus (HCV) oder für Epitope oder immunogene Abschnitteder genannnten HCV-Strukturantigene kodiert oder die hieraus transkribierte RNA.

## Claims

1. A recombinant MVA,
**characterized in that**
it contains DNA sequences coding for HCV structural antigens of the capsid protein core and/or the envelope glycoprotein E1 and/or the envelope glycoprotein E2 of Hepatitis C virus (HCV), for immunogenic portions or for epitopes of the said HCV structural antigens.

2. The recombinant MVA according to claim 1,
**characterized in that**
it contains as DNA sequences the sequences coding for E1 and E2.

3. The recombinant MVA according to claim 1 or 2,
**characterized in that**
the DNA sequences coding for E2 encode a mutated form of E2.

4. The recombinant MVA according to claim 3,
**characterized in that**
the mutated form of E2 is a secretable form in which at least a part of the lipophilic portion of E2 is deleted.

5. The recombinant MVA according to one or more of the preceding claims,
**characterized in that**
the DNA sequences are integrated into non-essential regions in the MVA genome.

6. The recombinant MVA according to one or more of the preceding claims,
**characterized in that**
the DNA sequences are integrated into portions of naturally occurring deletions in the MVA genome.

7. The recombinant MVA according to claim 6,
**characterized in that**
the site of the naturally occurring deletion is deletion III or another deletion in a non-essential region of the MVA genome.

8. The recombinant MVA according to one or more of the preceding claims,
**characterized in that**
the DNA sequences are under transcriptional control of vaccinia virus specific promotors and/or under control of promotors which are not derived from vaccinia virus, preferably in connection with further control sequences, such as enhancer elements.

9. The recombinant MVA according to claim 8,
**characterized in that**
the DNA sequences are under transcriptional control of the vaccinia virus specific Early/Late promotor P7.5.

10. The recombinant MVA according to one or more of the preceding claims,
**characterized in that**
E1 and E2 are not able to form heterodimers.

11. A pharmaceutical composition,
**characterized in that**
it contains at least one recombinant MVA according to one or more of the preceding claims 1-10 and pharmaceutically acceptable carriers and adjuvants.

12. The pharmaceutical composition according to claim 11,
**characterized in that**
it is present in the form of a vaccine.

13. Isolated eucaryotic cell,
**characterized in that**
it contains a recombinant MVA according to one or more of claims 1-10.

14. Eucaryotic cell according to claim 13
**characterized in that**
the cell is a chicken embryo fibroblast cell or a baby hamster kidney cell, preferably BHK-21, or an antigen presenting cell, preferably a dendritic cell.

15. The use of recombinant MVA according to one or more of claims 1-10 for the preparation of a medicament for therapy and prophylaxis of HCV infections and diseases caused thereby, in particular chronical liver diseases and liver tumours.

16. Use of recombinant MVA according to claim 15 for the preparation of a medicament contanting additionally at least pharmaceutically acceptable carrier and auxiliary substances.

17. The use of recombinant MVA according to one or more of claims 1-10 for the preparation of a vaccine, for the production of recombinant HCV structural proteins or for the preparation of eucaryotic cells producing the recombinant HCV structural protein.

18. A method for the preparation of recombinant HCV structural polypeptides or functional portions thereof, comprising the following steps of:
(a) cultivating cells according to claim 13 or 14 under suitable conditions; and
(b) expressing, isolating and optionally purifying recombinant HCV structural polypeptides, immunogenic portions thereof or epitopes thereof.

19. A method for the preparation of recombinant modified Vaccinia Virus Ankara (MVA) with the following steps of:
(a) introducing DNA sequences coding for HCV structural antigens of the capsid protein core and/or the envelope glycoprotein E1 and/or the envelope glycoprotein E2 of Hepatitis C virus (HCV), or for immunogenic protions or for epitopes thereof in a non-essential region of a MVA vector for the preparation of a recombinant MVA vector;
(b) introducing the recombinant MVA vector into a eucaryotic cell and amplifying the vector in said cell; and
(c) optionally isolating virus particles or DNA or RNA thereof.

20. DNA, or RNA transcribed thereof, of the recombinant MVA according to one or more of the preceding claims 1-10, preferably in isolated form, wherein said DNA or said RNA comprises said recombinant MVA, including the DNA, coding for the HCV structural antigens of the capsid protein core and/or the envelope glycoprotein E1 and/or the envelope glycoprotein E2 of Hepatitis C virus (HCV) or for epitopes or immunogenic portions of said HCV structural antigens or RNA transcribed thereof.

## Revendications

1. Virus de la vaccine Ankara modifié (MVA) recombinant, **caractérisé en ce qu'**il contient des séquences d'ADN qui codent pour les antigènes structuraux du virus de l'hépatite C de la protéine de capside gore et/ou de la glycoprotéine d'enveloppe E1 et/ou de la glycoprotéine d'enveloppe E2 du virus de l'hépatite C (HCV), pour des segments immunogènes ou pour des épitopes desdits antigènes structuraux de HCV.

2. MVA recombinant selon la revendication 1, **caractérisé en ce qu'**il contient en tant que séquences d'ADN les séquences codant pour E1 et E2.

3. MVA recombinant selon la revendication 1 ou 2, **caractérisé en ce que** les séquences d'ADN codant pour E2 codent pour une forme mutée de E2.

4. MVA recombinant selon la revendication 3, **caractérisé en ce que** la forme mutée de E2 est la forme pouvant être sécrétée, dans laquelle est supprimée au moins une partie de la partie lipophile de E2.

5. MVA recombinant selon une ou plusieurs des revendications précédentes, **caractérisé en ce que** les séquences d'ADN sont intégrées dans des régions non essentielles dans le génome du MVA.

6. MVA recombinant selon une ou plusieurs des revendications précédentes, **caractérisé en ce que** les séquences d'ADN sont intégrées dans des régions de délétions apparaissant naturellement dans le génome du MVA.

7. MVA recombinant selon la revendication 6, **caractérisé en ce que** la position de la délétion apparaissant naturellement est la délétion III ou une autre délétion dans une région non essentielle du génome du MVA.

8. MVA recombinant selon une ou plusieurs des revendications précédentes, **caractérisé en ce que** les séquences d'ADN sont sous le contrôle transcriptionnel de promoteurs spécifiques du virus de la vaccine et/ou sous le contrôle de promoteurs non propres au virus de la vaccine, de préférence conjointement avec d'autres séquences régulatrices telles que des éléments activateurs.

9. MVA recombinant selon la revendication 8, **caractérisé en ce que** les séquences d'ADN sont sous le contrôle transcriptionnel du promoteur précoce/tardif P7.5 spécifique du virus de la vaccine.

10. MVA recombinant selon une ou plusieurs des revendications précédentes, **caractérisé en ce que** E1 et E2 ne sont pas aptes à la formation d'hétérodimères.

11. Préparation pharmaceutique, **caractérisée en ce qu'**elle contient au moins un MVA recombinant selon une ou plusieurs des revendications 1-10 précédentes et des véhicules et adjuvants pharmaceutiquement acceptables.

12. Préparation pharmaceutique selon la revendication 11, **caractérisée en ce qu'**elle se trouve sous forme d'un vaccin.

13. Cellule eucaryote isolée, **caractérisée en ce qu'**elle contient un MVA recombinant selon une ou plusieurs des revendications 1 à 10.

14. Cellule eucaryote selon la revendication 13, **caractérisée en ce que** la cellule est un fibroblaste d'embryon de poulet ou une cellule rénale de hamster nouveau-né, de préférence BHK-21, ou une cellule présentatrice de l'antigène, de préférence une cellule dendritique.

15. Utilisation d'un MVA recombinant selon une ou plusieurs des revendications 1 à 10, pour la fabrication d'un médicament destiné à la thérapie et la prophylaxie d'infections à HCV et de maladies provoquées par celles-ci, en particulier d'hépatopathies chroniques et de tumeurs du foie.

16. Utilisation d'un MVA recombinant selon la revendication 15, pour la fabrication d'un médicament contenant en outre au moins des véhicules et adjuvants pharmaceutiquement acceptables.

17. Utilisation d'un MVA recombinant selon une ou plusieurs des revendications 1 à 10, pour la fabrication d'un vaccin, pour la production de protéines structurales de HCV recombinantes ou pour la production de cellules eucaryotes produisant la protéine structurale de HCV recombinante.

18. Procédé pour la production de polypeptides structuraux de HCV recombinants ou de fragments fonctionnels de ceux-ci, comportant les étapes suivantes :
(a) culture de cellules selon la revendication 13 ou 14, dans des conditions appropriées, et
(b) expression, isolement et éventuelle purification des polypeptides structuraux de HCV recombinants, de segments immunogènes de ceux-ci ou d'épitopes de ceux-ci.

19. Procédé pour la production de virus de la vaccine Ankara modifié (MVA) recombinant, comportant les étapes suivantes :
(a) introduction de séquences d'ADN qui codent pour les antigènes structuraux pour la protéine de capside gore et/ou la glycoprotéine d'enveloppe E1 et/ou la glycoprotéine d'enveloppe E2 du virus de l'hépatite C (HCV), ou pour des segments immunogènes ou des épitopes de ceux-ci dans une région non essentielle d'un vecteur MVA pour la production d'un vecteur MVA recombinant ;
(b) introduction du vecteur de MVA recombinant dans une cellule eucaryote et multiplication du vecteur dans cette cellule ; et
(c) éventuellement isolement des particules virales ou de leur ADN ou ARN.

20. ADN, ou ARN transcrit à partir de celui-ci, du MVA recombinant selon une ou plusieurs des revendications 1-10 précédentes, de préférence sous forme isolée, l'ADN ou l'ARN comprenant le MVA recombinant, y compris l'ADN qui code pour les antigènes structuraux de HCV de la protéine de capside gore et/ou de la glycoprotéine d'enveloppe E1 et/ou de la glycoprotéine d'enveloppe E2 du virus de l'hépatite C (HCV), ou pour des segments immunogènes ou des épitopes desdits antigènes structuraux de HCV, ou l'ARN transcrit à partir de celui-ci.
